(19) Europäisches Patentamt
European Patent Office
Office européen des brevets

(11) EP 3 233 044 B1

(12) # EUROPEAN PATENT SPECIFICATION

(45) Date of publication and mention of the grant of the patent:
**02.10.2019 Bulletin 2019/40**

(21) Application number: **15820982.5**

(22) Date of filing: **18.12.2015**

(51) Int Cl.:
***A61K 8/73*** (2006.01) ***A61K 8/02*** (2006.01)
***A61K 8/11*** (2006.01) ***A61Q 9/00*** (2006.01)
***A61Q 13/00*** (2006.01) ***A61Q 19/00*** (2006.01)
***A61K 8/81*** (2006.01) ***A61K 8/27*** (2006.01)
***A61K 8/67*** (2006.01) ***A61K 8/891*** (2006.01)

(86) International application number:
**PCT/US2015/066658**

(87) International publication number:
**WO 2016/100809 (23.06.2016 Gazette 2016/25)**

(54) **ANHYDROUS COSMETIC COMPOSITION FOR TOPICAL APPLICATION IN INTIMATE AREAS**

WASSERFREIE KOSMETISCHE ZUSAMMENSETZUNG ZUR TOPISCHEN ANWENDUNG IM INTIMBEREICH

COMPOSITION COSMÉTIQUE ANHYDRE POUR APPLICATION TOPIQUE DANS LA ZONE INTIME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2014 US 201462093670 P**
**18.12.2014 US 201462093678 P**
**06.11.2015 US 201562251786 P**

(43) Date of publication of application:
**25.10.2017 Bulletin 2017/43**

(73) Proprietor: **The Procter & Gamble Company**
**Cincinnati, OH 45202 (US)**

(72) Inventors:
- **SCAVONE, Timothy, Alan**
  **Cincinnati, Ohio 45202 (US)**
- **AVILES, Misael, Omar**
  **Cincinnati, Ohio 45202 (US)**
- **DUVAL, Dean, Larry**
  **Cincinnati, Ohio 45202 (US)**
- **ELLINGSON, Peter, Christopher**
  **Cincinnati, Ohio 45202 (US)**
- **GRAY, Brian, Francis**
  **Cincinnati, Ohio 45202 (US)**
- **KARAPASHA, Nancy**
  **Cincinnati, Ohio 45202 (US)**
- **STONE, Keith, Joseph**
  **Cincinnati, Ohio 45202 (US)**
- **WARREN, Raphael**
  **Cincinnati, Ohio 45202 (US)**
- **BRESLIN, Nery, Vanesa**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-00/74643** **WO-A2-2008/104960**
**DE-A1-102008 035 013** **US-A1- 2004 001 891**
**US-A1- 2008 213 204** **US-B1- 6 258 346**
**US-B1- 6 893 647**

EP 3 233 044 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### FIELD OF THE INVENTION

The present invention relates to cosmetic compositions for application to the skin, in particular to the skin of the intimate area.

### BACKGROUND OF THE INVENTION

Cosmetic compositions for application to the skin are known. Moisturizers, deodorants, antiperspirants are all commonly used ingredients for such compositions. Typically skin care compositions are in the form of thick creams which are dispensed from tubes or bottles, but in the antiperspirant area are also common in the form of creams extruded from an applicator container, roll-on liquids, solid sticks, or sprays.

WO00/74643 discloses skin care sticks, which are used for diapered skin. Despite the large number of products available in the market, there is still a need for a solution to everyday intimate hygiene and skin care especially for women: women have special needs in the intimate area including the need for discreet intimate odor protection against vaginal odor and odor from sporadic unpredictable vaginal discharges that is particularly effective when these discharges occur. Moreover, an increasing trend toward intimate grooming has increased the demand for preventing irritation from hair cutting or removal in the intimate area. For example, shave bumps or ingrown hairs following hair removal and intimate itch as hair grows back following removal, as well as the need to facilitate healing of small cuts and/or burns in the skin from intimate shaving can result from hair cutting / hair removal.

Additionally, many women facing problems with urine and vaginal discharge leakage also need protection from skin rash that occurs from wearing an absorbent article all day that rubs against bare, overly hydrated skin.

Currently, there is no product that simultaneously addresses these problems for women in the female intimate area in a form that is easy to use and apply, which is dry on application, and which does not leave greasy stains on clothes. The present invention aims at addressing this need in particular through the usage of an anhydrous composition.

### SUMMARY OF INVENTION

The present invention relates to an anhydrous cosmetic composition for topical application to the intimate skin area in conjunction with one or both of shaving pubic hair and managing incontinence wherein absorbent articles are worn against the body for long periods of time. The scope of the invention is defined by the appended claims.

In the present invention, an anhydrous cosmetic composition for topical application comprises: one or more complexed or encapsulated compounds having a thiol vapor pressure suppression index (TVPS) of more than 20, selected from the list of compounds defined in claim 1. The TVPS is measured according to the test methodology described herein. The anhydrous cosmetic composition may be in the form of a solid stick comprising a product hardness of at least 600 gram-force and devoid of an antiperspirant active.

In some forms, an anhydrous cosmetic composition comprises: a volatile solvent comprising a siloxane; a thickening agent; and a cosmetic active selected from the group consisting of zinc oxide and an oil-soluble Vitamin B5 derivative. The anhydrous cosmetic composition may be in the form of a solid stick comprising a product hardness of at least 600 gram-force and devoid of an antiperspirant active.

In some forms, a method of grooming hair in the intimate skin area is provided. The method comprising the steps of: cutting or removing hair from skin in the intimate skin area by at least one of the following methods: shaving, trimming, laser hair removal, epilation, waxing, chemical methods, and the like; thereafter, prepping the skin in the intimate skin area for application of a cosmetic composition; and thereafter, rubbing an anhydrous cosmetic composition onto the skin in the intimate skin area, the anhydrous cosmetic composition comprising: a volatile solvent; and a product hardness of at least 600 gram-force.

In some forms, a method of managing incontinence is provided. The method comprising the steps of: rubbing an anhydrous cosmetic composition onto skin and/or hair in an intimate skin area to apply the anhydrous cosmetic composition to the same, the anhydrous cosmetic composition comprising: a product hardness of at least 600 gram-force; and a volatile solvent; and applying an incontinence absorbent article against the skin and/or hair in the intimate skin area comprising the anhydrous cosmetic composition, the incontinence absorbent article comprising a liner, a pad, or a pant.

### BRIEF DESCRIPTION OF THE DRAWING

Fig. 1 is a perspective view of an absorbent article in the form of a pant.

DETAILED DESCRIPTION OF THE INVENTION

The term "anhydrous" as used herein means that the composition of the present invention are preferably substantially free of water. From a formulation standpoint, this means that the compositions of the present invention preferably contain less than about 2%, preferably less than about 1%, more preferably less than about 0.5%, most preferably zero percent, by weight of water.

The term "intimate area" as used herein means the area between the waist and the upper thigh (e.g., the pelvic and pubis region). The compositions of the present invention can be employed for management of hair and skin associated with the pubis region.

The present invention relates to an anhydrous cosmetic composition for topical application to skin in the intimate area. The cosmetic compositions can contain a number of different ingredients, including reactive compounds, encapsulated reactive compounds, complexed compounds, cosmetic actives, a formulation raw materials. These ingredients are described in greater detail below.

Examples of preferred reactive compounds suitable for use in the present invention are: melonal, adoxal, trans-2-hexenal, ligustral, Floral Super, Florhydral, 5-methyl-2-thiophenecarboxaldehyde, hydratropic aldehyde, undecenal, 9-undecenal, 10-undecenal, trans-4-decenal, cis-6-nonenal, isocyclocitral, precyclemone b, (E)-2-(z)-6-nonadienal, undecyl aldehyde, methyl-octyl-acetaldehyde, Lauric aldehyde, silvial, vanillin, floralozone.

Encapsulated compounds

The reactive compounds of the present invention can be encapsulated using any technique known in the art. The term "Encapsulation" within the present invention is intended to encompass any technology which allows introducing a reactive compound according to the invention into a composition according to the invention in a mixture with other materials which are called in general "encapsulating materials". The reactive compounds when encapsulated are prevented from contacting other materials so to avoid unwanted reactions. Moreover, when encapsulated, their evaporation is prevented. Many types of capsules are known in the art and are used for the delivery of perfume ingredients. All these types of capsules are usable in the present invention. Capsules can have any size, typically used in the art and suitable herein are nanocapsules, microcapsules, and larger capsules. In general capsules will have a size such that their shorter diameter will be lower than 3 mm or lower than 1mm.

Capsules allow the encapsulated composition to release when it is needed. Typically in the case of the present invention this corresponds to two cases:

1-when the skin receives a liquid insult (e.g. when urine, menses, sweat or vaginal fluids are discharged): in this case capsules comprise water soluble materials or materials which trigger release of the encapsulated compound when contacted with water or a water containing liquid.

2-when pressure or force is exerted on the capsules, e.g., during application of the topical composition or later once the composition is applied onto the skin due to normal movement, abrasion between the thighs or between the underwear and the skin. In this case, for example, breakable capsules having a shell of rupturable polymeric film can be used.

All these types of capsules are known in the art, e.g., as perfume delivery systems. These two cases should however be intended as non-limiting examples. In fact, any other trigger (or combination of triggers) can be used to release the encapsulated compound from the capsule, e.g. evaporation, diffusion, temperature, humidity, light etc. The release of the encapsulated compound can be instantaneous or sustained over time, depending on needs. The skilled person, based on the desired trigger action and release type, will be able to select the appropriate encapsulating material from those known in the art.

Capsules can use different encapsulating materials:

I. Polymers. Polymeric materials can be used as encapsulating materials.

Classical coacervates, water soluble or partly soluble to insoluble charged or neutral polymers, liquid crystals, hot melts, hydrogels, perfumed plastics, microcapsules, nano- and micro-latexes, polymeric film formers, and polymeric absorbents, polymeric adsorbents, etc. are some examples. Polymeric capsules include but are not limited to:

a.) Matrix Systems: The compound to be encapsulated is dissolved or dispersed in a polymer matrix or particle. Such compounds, for example, may be dispersed into the polymer prior to formulating into the product. Diffusion of the encapsulated compound from the polymer is a common trigger that allows or increases the rate of compound release from a polymeric matrix system that is deposited or applied to the desired surface, although many other triggers are known that may control compound release. Absorption and/or adsorption into or onto polymeric particles, films, solutions, and the like are aspects of this technology. Nano- or micro-particles composed of organic materials

(e.g., latexes) are examples. Suitable particles which can be used herein include a wide range of materials including, but not limited to polyacetal, polyacrylate, polyacrylic, polyacrylonitrile, polyamide, polyaryletherketone, polybutadiene, polybutylene, polybutylene terephthalate, polychloroprene, poly ethylene, polyethylene terephthalate, polycyclohexylene dimethylene terephthalate, polycarbonate, polychloroprene, polyhydroxyalkanoate, polyketone, polyester, polyethylene, polyetherimide, polyethersulfone, polyethylenechlorinates, polyimide, polyisoprene, polylactic acid, polymethylpentene, polyphenylene oxide, polyphenylene sulfide, polyphthalamide, polypropylene, polystyrene, polysulfone, polyvinyl acetate, polyvinyl chloride, as well as polymers or copolymers based on acrylonitrile-butadiene, cellulose acetate, ethylene-vinyl acetate, ethylene vinyl alcohol, styrene-butadiene, vinyl acetate-ethylene, and mixtures thereof.

"Standard" systems refer to those that are "pre-loaded" with the intent of keeping the pre-loaded compound associated with the polymer until the moment or moments of release. Such polymers may also suppress the neat product odor and provide a bloom and/or longevity benefit depending on the rate of compound release. One challenge with such systems is to achieve the ideal balance between 1) in-product stability (keeping the compound inside carrier until you need it) and 2) timely release (during use). Suitable micro-particles and micro-latexes as well as methods of making same may be found in USPA 2005/0003980 A1. Matrix systems also include hot melt adhesives and perfumed plastics. Polymer Assisted Delivery (PAD) matrix systems may include those described in the following references: US Patent Applications 2004/0110648 A1; 2004/0092414 A1; 2004/0091445 A1 and 2004/0087476 A1; and US Patents 6,531,444; 6,024,943; 6,042,792; 6,051,540; 4,540,721 and 4,973,422.

Silicones are also examples of polymers that may be used as encapsulating materials and can provide compound release benefits. Suitable silicones as well as making same may be found in WO 2005/102261; USPA 20050124530A1; USPA 20050143282A1; and WO 2003/015736. Functionalized silicones may also be used as described in USPA 2006/003913 A1. Examples of silicones include polydimethylsiloxane and polyalkyldimethylsiloxanes.

b.) Reservoir Systems: Reservoir systems are also known as a core-shell type technology, in which the compound to be released is surrounded by a release controlling membrane, which serves as a protective shell. The material inside the capsule is referred to as the core, internal phase, or fill, whereas the wall is sometimes called a shell, coating, or membrane. Depending on the type of shell materials the capsules can be activated by different mechanisms, for example the coating can be soluble in water or soluble in water solutions having a certain pH. In certain embodiments of the present invention the reservoir capsules have water insoluble shells and the core of the capsule is released upon mechanical activation.

Pressure sensitive capsules or friable capsules are examples of this technology. Friable capsules can be made in any sizes, and shapes, typically used are friable microcapsules. Any type of polymeric material can be used to make the shell of friable capsules, as well as any material can be used as a core material as known in the art. A skilled person will be able to determine which materials can be used to encapsulate certain core materials based on the knowledge available in the art concerning the compatibility of the materials (e.g. in general the shell material is selected so that core material will not act as a solvent on it). Friable microcapsules will be described now in more detail, it is clear to the skilled person that the same type of materials and construction can be used to make larger or smaller capsules. Friable microcapsules are capsules where the outer shell is made from any polymer or mixture of polymers. Typical polymers which can be used to be comprised in the shell of a friable microcapsule include melamine-formaldehyde or urea-formaldehyde condensates, melamine-resorcinol or urea-resorcinol condensates, nylon, polyacrylates, polyethylenes, polyamides, polyamides, polystyrenes, polyisoprenes, polycarbonates, polyesters, polyureas, polyurethanes, polyolefins, polysaccharides, epoxy resins, vinyl polymers, silk, wool, gelatin, cellulose, proteins and mixture thereof as well as co-polymers comprising, as co monomers, monomers contained in these mentioned polymers.

Among the most stable friable microcapsules are those comprising polyoxymethyleneurea (PMU)-based polymers, melamine-formaldehyde based polymers, and polyacrylate based polymers.

In some embodiments the microcapsule outer shell material can include a polyacyrylate material. Any polymer or copolymer including acrylate or metacrylate monomers can be used in the present invention, preferred materials are those known in the art as forming polyacrylate microcapsules such as, for example, those described in US2012-276210A1. In some embodiments the shell of the microcapsules comprises a polyacrylate copolymer, in some cases this can be a polyacrylate random copolymer.

A friable microcapsule is configured to release its core substance when its outer shell is ruptured. The rupture can be caused by forces applied to the outer shell during mechanical interactions. Friable microcapsules can have various fracture strengths. Each microcapsule can have an outer shell with a fracture strength of 0.2-10.0 mega Pascals, when measured according to the Fracture Strength Test Method, described in co-pending application US 61/703587. As an example, a microcapsule can have an outer shell with a fracture strength of 0.2-2.0 mega Pascals.

Friable microcapsules can have various core to outer shell ratios. Each microcapsule has an outer shell, and

a core within the outer shell, and a core to outer shell ratio (in weight) from 99-1 to 1-99, or from 95-5 to 10-90, or from 50-50 to 90-10. Friable microcapsules can have various outer shell thicknesses. In some forms the microcapsule can have an outer shell with an overall thickness of 1-300 nanometers or 2-200 nanometers.

In the present invention it is especially preferred that the microcapsule is introduced as an anhydrous particle. Such particles may be produced by spray drying as described in U.S. patent application serial no. 61/703616. In the instances where friable microcapsules are spray dried, it is preferable to introduce these particles in the compositions of the invention in a paste or slurry comprising a carrier vehicle.

Friable microcapsules and relative methods for making them as well as methods to measure their properties which can be used herein are described in co-pending U.S. patent application serial nos. US 61/703616 and US 61/703587, which are incorporated herein by reference.

Example methods for making polyacrylate microcapsules are disclosed in U.S. Patent Application 61/328,949; U.S. Patent Application 61/328,954; U.S. Patent Application 61/328,962; and U.S. Patent Application 61/328,967 which are incorporated herein by reference.

II. Starches: The use of a starch encapsulation technology allows one to modify the properties of the compound to be encapsulated, for example, by converting a liquid compound into a solid by adding ingredients such as starch. The benefit includes increased retention for volatile compounds during product storage. Upon exposure to moisture, a release may be triggered. Another benefit is that the starch encapsulation allows the product formulator to select compounds or concentration of compounds that normally cannot be used without the presence of starch encapsulation. Suitable starch encapsulation examples as well as methods of making the same may be found in US 2005/0003980 A1 and US 6,458,754 B1.

In one aspect, starch encapsulated compounds may be made by preparing a mixture comprising starch, water, acid and the compound(s) which need to be encapsulated, the acid being incorporated in the mixture in an amount sufficient to lower the pH of the starch-water mixture by at least 0.25 units; and spray drying the mixture thereby forming the encapsulated compound(s). In the first step in the process of compound(s) encapsulation, an aqueous mixture is prepared comprising starch, water, the compound(s) which need to be encapsulated and acid. These ingredients may be added in any order, but usually the starch-water mixture is prepared first and subsequently, either sequentially or together, the acid and compound(s) to encapsulate are added. When they are added sequentially, the acid may be added prior to the ingredient for encapsulation. Alternatively, the acid is added after the ingredient for encapsulation. The concentration of starch in the aqueous mixture may be from as low as 5 or 10 wt% to as high as 60 or even 75 wt%. Generally the concentration of starch in the mixture is from 20 to 50 wt%, more usually around 25 to 40 wt% in the aqueous mixture.

Suitable starches can be made from raw starch, pregelatinized starch, modified starch derived from tubers, legumes, cereal and grains for example corn starch , wheat starch, rice starch, waxy corn starch, oat starch, cassava starch, waxy barley starch, waxy rice starch, sweet rice starch, amioca, potato starch, tapioca starch and mixtures thereof. Modified starches may be particularly suitable for use in the present invention, and these include hydrolyzed starch, acid thinned starch, starch having hydrophobic groups, such as starch esters of long chain hydrocarbons ($C_5$ or greater), starch acetates, starch octenyl succinate and mixtures thereof. In one aspect, starch esters, such as starch octenyl succinates are employed.

The term "hydrolyzed starch" refers to oligosaccharide-type materials that are typically obtained by acid and/or enzymatic hydrolysis of starches, preferably corn starch. It may be preferred to include in the starch water-mixture, a starch ester. Particularly preferred are the modified starches comprising a starch derivative containing a hydrophobic group or both a hydrophobic and a hydrophilic group which has been degraded by at least one enzyme capable of cleaving the 1,4 linkages of the starch molecule from the non-reducing ends to produce short chained saccharides to provide high oxidation resistance while maintaining substantially high molecular weight portions of the starch base. The aqueous starch mixture may also include a plasticizer for the starch. Suitable examples include monosaccharides, disaccharides, oligosaccharides and maltodextrins, such as glucose, sucrose, sorbitol, gum arabic, guar gums and maltodextrin.

The acid used in the process of the invention may be any acid. Examples include sulfuric acid, nitric acid, hydrochloric acid, sulfamic acid and phosphonic acid. In one aspect, carboxylic organic acids are employed. In another aspect, organic acids comprising more than one carboxylic acid groups are employed. Examples of suitable organic acids include citric acid, tartaric acid, maleic acid, malic acid, succinic acid, sebacic acid, adipic acid, itaconic acid, acetic acid and ascorbic acid, etc. In one aspect, saturated acids, such as citric acid, are employed.

Following the formation of the aqueous mixture comprising starch, water, perfumes and acid, the mixture is mixed under high shear to form an emulsion or dispersion of ingredient for encapsulation in the aqueous starch solution.

Any suitable technique may then be used for the final stage of processing where the aqueous mixture including acid and perfumes is atomized and dried. Suitable techniques include, but are not limited to those known in the art including spray drying, extrusion, spray chilling/crystallization methods, fluid bed coating and the use of phase transfer catalysts to promote interfacial polymerization. Spray efficiencies may be increased by methods known in the art, such

as by using high drying towers, lightly oiling the chamber walls, or using preconditioned air in which the moisture has been substantially removed.

Coated capsules

In some forms, the primary materials forming the capsule as described so far, may be further encapsulated with a secondary coating material. Any of the capsule types mentioned so far can be used in the present invention as such or with an additional secondary coating material.

An additional secondary coating material can help in reducing the scent perception, in reducing evaporation of volatile components over time (especially at elevated temperatures and humidity conditions) and in increasing chemical stability of the complexed compound by reducing the exposure of the complexed compounds (which in the present invention comprise highly reactive materials) to prematurely react or decompose so they are no longer functional or have a different odor character when activated. Additionally the use of coated capsules can allow altering the release characteristic of the encapsulated material (slowing or accelerating its release, or changing the release trigger, for example introducing a pH trigger). Generally, any second material that is added to or applied directly to a primary encapsulating material that accomplishes one or more of the above functions is characterized as a coating. The secondary coating may be directly applied using a second process step following creation of the primary capsule, using a process such as prilling, or using any fluidized bed process to apply a secondary surface coating (for example a Wurster Coater).

Coating compositions which are suitable for the present invention are all capsule coating compositions which are commonly known in the art. These include for example: polysaccharides (for example, but not limited to unmodified starch, chemically modified starch, dextrins, cyclodextrin and cyclodextrin derivatives), natural and artificial/synthetic waxes, esters and ester derivatives, fatty acids, natural and synthetic and chemically modified lipids, fatty alcohols, hydrocarbons (linear or branched, petrolatum), enteric coating compositions (such as the Eudragit series of Methacrylic acid co-polymers), polyvinyl alcohols, polyethylene glycols, silicones (for example, but not limited to silicone copolymers and functionalized silicones), surfactants, emulsifiers, polypropylene glycols, cellulose derivatives (methyl cellulose, hydroxypropyl cellulose), glycerin, mono and diglycerides, polyglycerol and polyglycerol esters and emulsifiers employed in food applications.

An example of the preparation of a coated capsule which can be used in the present invention has been described in US4973422 (see in particular Example 2).

Complexed compounds

For "complex" it is intended an "inclusion complex" within the meaning of IUPAC Compendium of Chemical Terminology 2nd Edition (1997) wherein the complexing agent is the host and the complexed compound is the "guest". Examples of complexing agents are cyclodextrins. As used herein, the term "cyclodextrin" includes any of the known cyclodextrins such as substituted and unsubstituted cyclodextrins containing from about six to about twelve glucose units, for example alpha-cyclodextrin, beta-cyclodextrin, gamma-cyclodextrin and/or their derivatives and/or mixtures thereof. For example, the cyclodextrin complex of the present invention can comprise cyclodextrin selected from the group consisting of beta-cyclodextrin, alpha-cyclodextrin, hydroxypropyl alpha-cyclodextrin, hydroxypropyl beta-cyclodextrin, methylated-alpha-cyclodextrin, methylated-beta-cyclodextrin, and mixtures thereof. Cyclodextrin complexes of compounds which are active against malodors can be prepared as known in the art for example using the kneading method described in US 5,571,782 and US 5,543,157 or, preferably, using the spray drying method described in WO2008/104690A2.

Thiol Vapour Pressure Suppression Index

In some forms, the present invention relates to compositions that may comprise one or more complexed or encapsulated compounds having a "thiol vapor pressure suppression index" (TVPS) of more than 20.

Thiol Vapor Pressure Suppression (TVPS) index is a measure of the reduction in butanethiol concentration in the headspace by a compound, as measured using a fast GC instrument, the zNose 7100 (Electronic Sensor Technologies, Newbury Park, CA). Before any measurements the instrument is calibrated according to manufacturer's instructions under the same experimental settings. The instrument has a DB-5 column (EST Part No.SYS7100C5, Electronic Sensor Technologies, Newbury Park, CA) 1 m in length, 0.25 μm phase thickness, and 0.25mm in diameter. The experimental settings for TVPS measurements are:

Sampling time: 10s
Sensor Temperature: 40 °C
Initial Column Temperature: 40 °C

Inlet Temperature: 40 °C
Valve Temperature: 40 °C
Column Temperature Ramp Rate: 10 C°/s
Final Column Temperature: 200 °C

TVPS of a compound is measured in the following way:
100 μl ± 1 μl of a 1% v/v butanethiol (99%, purity) solution in ethanol (200 proof) is added into a 1 ml vial (8 x 40 mm). These vials are borosilicate glass straight walled vial. A suitable butanethiol is item 112925 from Sigma-Aldrich (Sigma-Aldrich, St. Louis, MO). In another 1 ml vial (8 x 40 mm), 5 μl± 0.2μl of the compound is added. Both open vials are then placed inside a 20 ml headspace vial (22 x 75 mm), and the vial is immediately sealed using a screw thread closure with PTFE/Silicone septa. The vial is heated to 37 °C for 4 hours. After 4 hours, the vial is removed from the oven and let to equilibrate at 25 °C ± 2 °C for 15 minutes. The headspace inside the vial is sampled using the zNose following the experimental protocol outlined above. Samples with butanethiol alone, and the volatile active alone, are run using the same protocol to identify the peaks for both materials. An acceptable retention index for butanethiol is 720±30. If the butanethiol peak and the volatile material peak co-elute, one skilled in the art can modify the protocol settings to separate those peaks. A minimum resolution of 1.5 should be obtained. For example one can change the column temperature ramp rate. In between samples, the instrument needs to be cleaned to remove any trace materials. To clean the instrument, the instrument is run without samples as needed until no peaks greater than 100 counts are observed.

The amount of butanethiol in the headspace is measured from the area of the peak on the chromatograph for butanethiol ($A_{BtSH,Rx}$). To calculate the percentage of butanethiol reduction in the headspace, a control with the butanethiol solution without the volatile material is run in the same manner and the area is measured as well ($A_{BtSH,C}$). TVPS is then measured as the percentage reduction in butanethiol area calculated using the following formula:

$$TVPS = \frac{A_{BtSH,C} - A_{BtSH,Rx}}{A_{BtSH,C}} \times 100$$

An example of the type of measurements obtained with the instrument is:

| Sample | Butanethiol Peak Retention Index | Area (counts) |
|---|---|---|
| Butanethiol Control<br>Vial 1: 100 μl of 1% v/v butanethiol in ethanol<br>Vial 2: Empty | 720 | $A_{BtSH,C}$ = 4934 |
| Butanethiol + Florhydral<br>Vial 1: 100 μl of 1 % v/v butanethiol in ethanol<br>Vial 2: 5 μl Florhydral | 720 | $A_{BtSH,Rx}$ = 2442 |

Example TVPS calculation for

$$TVPS = \frac{4934 - 2442}{4934} \times 100 = 50.5$$

The value of TVPS for several compounds suitable for the invention is presented in the table below. TVPS for the compounds indicated with (*) have been approximated using a mathematical model calculated starting from real measurements on a large number of compounds. The model is created using the QSAR software CAChe ProjectLeader WorkSystem Pro 7.1. Using the molecular structure from the compounds for which TVPS was evaluated, several molecular properties are calculated. A regression algorithm is the used to calculate the best fit to predict TVPS based on the 4 molecular descriptors that best fit the data. The model is then used to predict TVPS for other compounds using the same software. The values of TVPS approximated with the molecular modeling system are presented for illustration only, for the avoidance of doubt it is specified that the TVPS values for use in the present inventions are only the TVPS values measured with the zNose analytical method described above.

Compounds indicated as (**) indicate prior art compounds.

| | TVPS |
|---|---|
| melonal | 20.4 |
| adoxal | 24.4 |
| trans-2-hexenal | 27.1 |
| ligustral | 42.5 |
| Floral Super | 52.4 |
| Florhydral | 53.3 |
| 5-methyl-2-thiophene-carboxaldehyde | 67.4 |
| hydratropic aldehyde(*) | 72.0 |
| Undecenal(*) | 26.2 |
| 9-undecenal(*) | 67.5 |
| 10-undecenal(*) | 52.0 |
| trans-4-decenal(*) | 60.3 |
| cis-6-nonenal(*) | 57.1 |
| isocyclocitral(*) | 51.4 |
| precyclemone b(*) | 40.7 |
| (E)-2-(z)-6-nonadienal(*) | 35.8 |
| undecyl aldehyde(*) | 34.9 |
| methyl-octyl-acetaldehyde(*) | 30.2 |
| Lauric aldehyde(*) | 26.6 |
| silvial(*) | 25.8 |
| vanillin(*) | 23.7 |
| floralozone(*) | 23.5 |
| Hexylcinnamic aldehyde(**) | 8.0 |
| (**) neral | 17.1 |
| ethyl vanillin (**) | 2.9 |

As it can be seen, some of the compounds mentioned in the prior art as being very effective in general for the control of body fluids originated malodors, such as Hexylcinnamic aldehyde, have surprisingly low TVPS values. It is believed that such compounds, while very effective against some of the malodorant compounds such as those comprising ammonia or amine groups, are surprisingly less effective in counteracting other types of malodors such as those deriving from protein degradation and containing thiol groups. Reactive compounds according to the present invention, having relatively high TVPS values are surprisingly effective in counteracting both types of malodorant molecules and are therefore overall more effective in neutralizing malodors in a broader range of situations.

Examples of preferred compounds which are suitable for the present invention and which have a TVPS higher than 20 are those of the following list (a), these compounds not only have a TVPS higher than 20 but also they form complexes and/or capsules which are particularly stable and release the complexed encapsulated materials when needed. In particular these preferred materials form complexes with cyclodextrins in a complete manner. Forming complexes in a complete manner means in this context that a skilled person, using methods known in the art, can combine the compound with cyclodextrin and obtain a material where the wt% of compound which is complexed with the cyclodextrin is greater than about 75%, greater than about 90%, or greater than about 95%. These complexes are very stable over time and the complexed compound gets released very quickly when the complex gets in contact with a water based fluid. (a): melonal, adoxal, trans-2-hexenal, ligustral, Floral Super, Florhydral, 5-methyl-2-thiophenecarboxaldehyde, hydratropic aldehyde, undecenal, 9-undecenal, 10-undecenal, trans-4-decenal, cis-6-nonenal, isocyclocitral, precyclemone b, (E)-2,(z)-6-nonadienal, undecyl aldehyde, methyl-octyl-acetaldehyde, Lauric aldehyde, silvial, vanillin, floralozone.

All these compounds in list (a) are particularly reactive toward malodorant molecules containing Sulphur atoms (thiol type malodors, typically associated with protein degradation e.g. in menstrual fluids, feces, food etc). The primary function of the complexed or encapsulated reactive compounds is to chemically react with malodors, such as malodorant molecules containing Nitrogen atoms (amine type odors, typically deriving from the degradation of urine or certain foods like onions) and/or malodorant molecules containing Sulphur atoms (thiol type malodors, typically associated with protein degradation, e.g., in menstrual fluids, feces, food etc). Ammonia/amines are one component of malodor associated with the absorption of bodily fluids, such as menses or urine. For example, ammonia/amines are typically present in high amounts in absorbent products used for urine absorption due to degradation of urea. Ammonia/amines and their derivatives can react with aldehydes and/or ketones to form imines (according to the so-called Schiff base reaction).

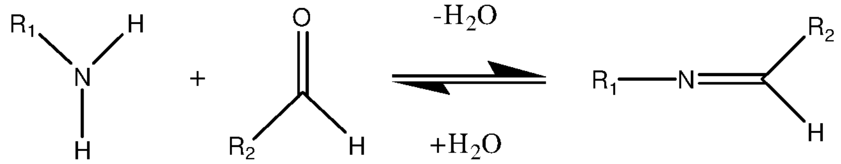

This reaction is catalyzed by enzymes and/or by a slightly acidic pH 4 to 5. The moderate acid requirement is necessary to allow protonation of the hydroxyl intermediate to allow water to leave.

Malodorant sulphur based compounds are typically generated by the degradation of proteins e.g. in menstrual fluids and feces so their control is particularly important in an intimate hygiene context. The mechanism of action is not fully understood at the moment, but it is believed that it is connected to the fact that Thiols can react with aldehydes and ketones to form thioacetals and thioketals.

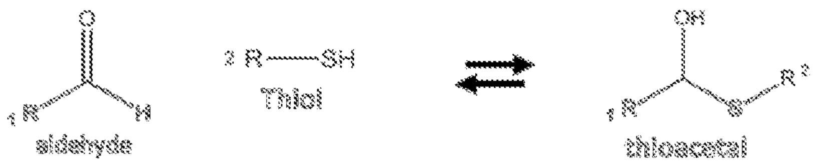


In principle, the chemical reactions described above can be obtained from any aldehyde, but in practice the reactivity of aldehydes in these type of reactions is very different. The reactive compounds of the present invention are effective in reacting with Nitrogen based malodorant molecules and particularly effective in reacting with sulphur based malodorant molecules.

The particularly high reactivity of the reactive compounds of the invention towards sulphur based malodorant molecules renders the present invention particularly effective for use in the intimate area.

In addition, the preferred reactive compounds of the present invention are particularly advantageous in the specific context of compositions for topical use because they have a pleasant and low intensity odor and are also able to be complexed or encapsulated effectively and to be quickly released when needed.

Another important aspect of the present invention is that each complexed or encapsulated reactive compound has an individual character in terms of odor. Therefore their introduction within a composition also represents the possibility to provide not only reactivity on malodors but also individual fragrant notes which can be combined with other odorous components (encapsulated/complexed and/or in free uncomplexed form) thus allowing the formulator to obtain a broader range of fragrances being released by the composition when used, i.e., when the encapsulated/complexed reactive compound is activated.

Additional compounds

In the present invention, other selected additional compounds in complexed or encapsulated form can be optionally used in combination with the new reactive compounds having a TVPS of more than 20, described above in list (a). Preferred additional compounds are listed here below in lists (b), (c), (d) and (e).

Suitable selected additional aldehydes and/or ketones include the following listed in list (b): hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, salycil aldehyde, citral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)-,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyrraldehyde, helional, lyral, 3-hexenal, safranal, veratraldehyde, 3-(2-furyl)acrolein, pyruvaldehyde, ethanedial, 1-(2,6,6-trimethyl-1-cyclohexenyl)pent-1-en-3-one; 4-(2,6,6-trimethyl-1-cyclohexen-1-yl)-3- Buten-2-one; 4-(2,6,6-trimethyl-2-cyclohexen-1-yl)-3-buten-2-one, 5-(2,6,6-Trimethyl-2-cyclohexen-1-yl)4-penten-3-one, (E)-4-(2,2-dimethyl-6-methylidenecyclohexyl)but-3-en-2-one.

Compounds in list (b) are additional aldehydes and/or ketones which are able to react with some classes of malodorant compounds and do not have unpleasant odor. One or more of these other selected aldehydes and/or ketones can be optionally used in complexed or encapsulated form in combination with those mentioned previously in list (a).

Other additional optional compounds can be present in complexed or encapsulated form. These include in particular other fragrance/masking/reacting components. In some embodiments at least part of the additional components are selected from the following lists (c), (d) and (e). Components from list (c) are menthol, menthyl acetate, menthyl lactate, menthyl propionate, menthyl butyrrate, menthone, mint terpenes, laevo-carvone, Cis-3-Hexenol & Cis-3-Hexenyl acetate, koavone and methyl dioxolan.

These are all compounds whose primary function is to mask malodors. This may occur through vapor pressure suppression of the malodor or by overwhelming the unpleasant malodor with the pleasant odor of the fragrance component. These materials, when used, may significantly reduce the ability to detect the malodors. The masking ability to hide malodors is possible due to the volatile nature of the materials selected, which are released from the complex or capsule in the composition for topical use and are then inhaled into the nose of a user, generally within somewhat close range, e.g., within about 0 to 10 meters of the person by normal breathing (although this should in no way be intended to limit the scope of the invention).

Components from class (d) are methyl-dihydrojasmonate, methyl jasmonate, eucalyptol, tetrahydro-linalool, Phenyl-Ethyl alcohol, Hexyl iso-butyrate, Linalyl acetate, Benzyl acetate, Benzyl alcohol, or mixtures thereof. These are volatile materials which are well complexed in particular when the complexing agent is a cyclodextrin and are release very quickly upon contact with a water based liquid. Their presence allows the topical composition to respond more quickly to an insult of malodorant substances by releasing compounds that have a good general masking effect against malodors, in particular, being very volatile, reduces the vapor pressure of other malodorant compounds slowing down their evaporation rate.

Other suitable malodor masking and fragrance components which can optionally be used in complexed or encapsulated form in combination with those of list (a) include those in the following list e):

e) camphor, p-menthane, limonene, cresol, linalool, myrcenol, tetra hydromyrcenol, di-hydromyrcenol, myrcene, citronellol, citronellyil derivatives, geraniol, geranyl derivatives, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, camphene, citronellal, hydroxycitronellal, ethyl maltol, methyl phenyl carbinyl acetate, dihydrocumarin, di-hydromyrcenyl acetate, geraniol, geranial, isoamylacetate, ethyl, and/or triethyl acetate, para-cresol, para-cymene, methyl abietate, , hexyl-2-methyl butyrate, hexyl-2-methyl butyrate , and mixtures thereof.

All the compounds mentioned within the present application, unless a specific isomeric form is specified, also include their isomeric forms, diastereomers and enantiomers.

It may be that, for certain components, the same component can be considered both a malodor reactive component, a malodor masking component, and/or a fragrance component.

In forms of the invention wherein one or more compound of list a) is present in combination with one or more optional compounds of lists (b), (c), (d) or (e), the capsule or complex can be prepared mixing all compounds together before preparing the capsule or complex, or, alternatively, capsules or granules of complex containing only one or only some of the compounds can be prepared separately and then mixed according to the desires dosages before introduction into the composition of the invention.

In some forms, the composition of the present invention, in addition to the components from lists a), b), c), d) and e) in complexed or encapsulated form may also include components from the same lists or other fragrance components in free form (i.e.s not complexed or encapsulated).

In the case of cyclodextrin complexes, the percent of components that are complexed with cyclodextrin is greater than about 75%, greater than about 90%, or greater than about 95%. It should be understood that these levels of component complexation are directly associated with the complex formation process itself; i.e. the percentages do not represent a formulation design of adding a first percentage of components via a cyclodextrin complex and adding a second percentage of neat components.

Cyclodextrin complexes can be formed by various methods which are well known in the art. For example, US 5,543,157, US 5,571,782, and WO2008/104690A2 describe methods of forming cyclodextrin complexes.

As one example of a method of forming a cyclodextrin complex, a solvent (e.g., water or an organic solvent suitable for the organic compound to be complexed), unloaded cyclodextrin particles, and the organic compound which need to be complexed can be placed into a container and then mixed for a period of time to permit loading of organic molecules into "cavities" of cyclodextrin molecules. The mixture may or may not be processed further; e.g., processed through a colloid mill and/or homogenizer. The solvent is then substantially removed from the resulting mixture or slurry to yield cyclodextrin complex particles, e.g. via spray drying. Different manufacturing techniques may however impart different particle/complex characterizations, which may or may not be desirable in the compositions of the invention, depending on the specific usage and conditions. In some embodiments the particles of cyclodextrin inclusion complexes have a low level of typically of less than about 20% by weight of the particles, or of less than about 10% by weight of the particles, or of less than about 6% by weight of the particles. Spray drying a slurry of inclusion complexes of cyclodextrin and organic compounds is one manufacturing technique capable of producing the cyclodextrin particles and cyclodextrin complexes having the above-noted, moisture levels. Cyclodextrin complexes can also be obtained using known techniques and an extrusion process (kneading) however the resulting material will in general contain a higher humidity and a lower complexation efficiency. Also US 2008/0213191 A1 from The Procter & Gamble Company provides a detailed overview of preferred techniques for preparing cyclodextrin complexes.

Accordingly, in some forms, the present invention relates to an anhydrous cosmetic composition for topical application comprising one or more complexed or encapsulated reactive compounds having a thiol vapor pressure suppression index (TVPS) of more than 20.

In addition to the reactive compounds the cosmetic compositions of the present invention may include ingredients which are common in anhydrous cosmetic compositions. In some forms, the anhydrous cosmetic composition may comprise cosmetic compositions in the absence of the reactive compounds disclosed herein.

For example the anhydrous cosmetic composition of the present invention can comprise, in addition to the reactive compound(s) :

a) from 0.01% to 60% of a cosmetic active
b) from 10% to 95% of a volatile solvent
c) from 2-45% of a thickening agent
f) less than 5%, preferably less than 1% more preferably less than 0.1% of propylene and dipropylene glycol.

<u>Cosmetic actives</u>

The anhydrous cosmetic composition of the present invention can comprise from about 0.01 % to about 60% by weight of one or more cosmetic actives. Suitable actives include any known or otherwise effective cosmetic active that is compatible with the essential ingredients of the cosmetic sticks of the present invention, or which do not otherwise unduly impair the product performance thereof.

Cosmetic actives suitable for use in the compositions of the present invention include moisturizers, emollients, perfumes or fragrances, skin conditioners, antiperspirants, antioxidants, vitamins, anti-wrinkle products, anti-itch products (e.g., antihistamines such as diphenhydramine, corticoid steroids such as hydrocortisone and anesthetics such as benzocaine), surfactants, pharmaceuticals, deodorants, colorants, pigments, sunscreens or other photo protectants, topical hormones such as estrogens, isoflavones such as genistein and daidzein, materials which provide a tactile sensation of heat or cold such as menthyl lactate, and any other inert or active material intended or otherwise suitable for topical application to the skin.

Non-limiting examples of cosmetic actives suitable for use herein are described in U.S. Pat. No. 6,001,377 (SaNogueira, Jr. et al.), U.S. Pat. No. 6,024,942 (Tanner et al.), u.s. Pat. No. 6,013,271 (Doughty et al.), and U.S. Pat. No. 6,013,270 (Hargraves et al.), U.S. Pat. No. 6,013,248 (Luebbe et al.) U.S. Pat. No. 5,976.514 (Guskey et al.), which descriptions are hereby incorporated herein by reference.

Specific examples of cosmetic actives suitable for use herein include deodorant actives perfumes and fragrances, antimicrobials (antibacterial, antifungal), steroidal anti-inflammatory materials (e.g., hydrocortisone), non-steroidal anti-inflammatory materials, vitamins and derivatives thereof (e.g., thiamin, riboflavin, niacin, pyridoxine, vitamin A, vitamin D, vitamin E, vitamin K), hydroxy and alpha-hydroxy acids (e.g., salicylic acid, citric acid), moisturizers (e.g., silicone and non-silicone), and the like.

Paticularly preferred cosmetic actives for use herein are Zinc Oxide, Petrolatum, dimethicone, Panthenyl Triacetate, Lactic Acid, Vitamin $B_3$ and derivatives, Vitamin $B_5$ and derivatives, green tea extract, hexamidine, polycarbophil, pectin, allantoin, hyaluronic acid, zinc pyrithione, zinc carbonate, stearyl alcohol and glycerin, amino peptide complexes, and mixtures thereof.

Derivatives of Vitamin $B_5$ are believed to improve the condition of skin and to accelerate the rate of wound

healing. This can be particularly advantageous when applied to intimate area skin that is irritated or cut during shaving. Liquid derivatives of Vitamin $B_5$ are also believed to soften hair. Over repeated uses, this can allow a consumer to experience an easier or closer shave, and can make the pubic hair less itchy when it grows back. Lastly, it is believed that derivatives of Vitamin $B_5$ can reduce the number and extent of shave bumps that occur from ingrown hair. These advantages appear to be more noticeable in the intimate area where sensitivity to itch, irritation, shave bumps, and hair regrowth is greater. Benefits can be noticeable in 1-2 days versus one week in the underarm. To be able to formulate Vitamin $B_5$ into anhydrous media it is helpful to derivatize the vitamin so that it is sufficiently soluble in the anhydrous carrier while still retaining bioavailability for would healing properties. In one approach the degree of substitution may be 1.5-3 where the substitution chemistry contains one or more carbons and the derivative remain liquid at 75°F (hair temperature) so that it can effectively plasticize and soften the hair shaft. Panthenyl triacetate is an exemplary Vitamin $B_5$ derivative that is useful in compositions of the present invention.

Additional considerations regarding exemplary Vitamin $B_5$ derivatives may apply. For example, in order to deliver Vitamin $B_5$ (or a derivative thereof) from an anhydrous, hydrophobic, glycol free and antiperspirant free cosmetic stick, a corresponding matrix soluble form or derivative of Vitamin $B_5$ must be used to achieve the desired benefits of hair softening, shave bump reduction and wound healing from shave cut damage. For example, if an incompatible or insoluble form of Vitamin $B_5$ is used, such as panthenol, this will not form a homogenous one phase solution when the ingredients are blended and heated together from the melt. The panthenol will fall out of solution and not form a uniform stick. If the formulation contains cosmetic powders, such as cyclodextrin fragrance complexes, in the formulation the panthenol will cause particle aggregation and create hard lumps with an undesirable cosmetic feel on application of the stick.

As such, the Vitamin $B_5$ derivative utilized should be soluble in the anhydrous, hydrophobic, glycol free and antiperspirant free matrix. As shown below this is achieved when $R_1$, $R_2$, $R_3$ contain at least one or more carbon atoms, where $R_1$, $R_2$, $R_3$ each contain one or more carbons, or where the derivative is panthenyl triacetate. Alternatively, suitable Vitamin $B_5$ derivatives may be described via cLogP values for the Vitamin $B_5$ derivative, where the cLog P value should be greater than about -1.0, preferably greater -0.6, and most preferably greater than 0.0. Where R1, R2, R3 contain one or more carbon atoms, where R1, R2, R3 each contain at least one carbon atom, or where the cLog P values are greater than -0.6, the derivative is oil soluble. The values for cLog P can be determined by the method which is provided in U.S. Patent No. 6,352,688, issued to Scavone et al., specifically in columns 8 and 9.

As shown in Table 1, some exemplary derivatives of Vitamin $B_5$ are provided some which are soluble and may be utilized in the anhydrous cosmetic composition of the present invention and some Vitamin $B_5$ derivatives which are not soluble and would not provide an acceptable consumer experience during use and may provide limited functionality.

Table 1

| Name | Chemical Structure | Number of R-Groups with Carbon Substitution | Solublility in hydrophobic Anhydrous, glycol free, antiperspirant free Stick formulations | cLog P |
|---|---|---|---|---|
| | 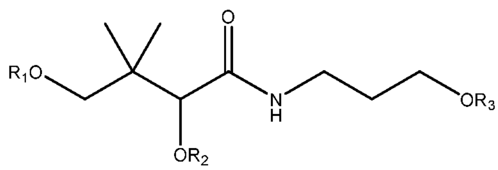 Base Chemical Structure (Available R-group substitution) | | | |
| Calcium Pantothenate | 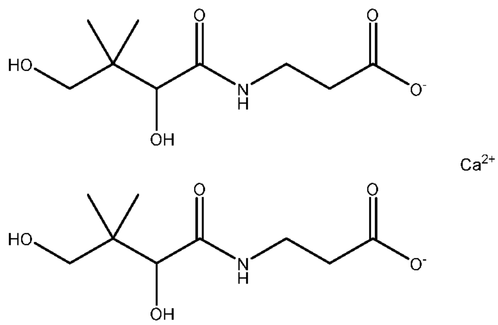 | 0 | Not Soluble Or Mixable | -4.454 |

(continued)

| Name | Chemical Structure | Number of R-Groups with Carbon Substitution | Solublility in hydrophobic Anhydrous, glycol free, antiperspirant free Stick formulations | cLog P |
|---|---|---|---|---|
| | 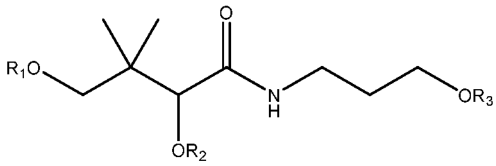 Base Chemical Structure (Available R-group substitution) | | | |
| Panthenol | 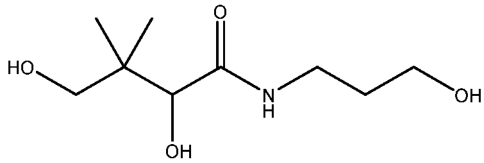 | 0 | Not Soluble or Mixable | -1.648 |
| Panthenyl Triacetate | | 3 | Soluble | 0.656 |
| Panthenyl Ethyl Ether | | 1 | Soluble | -0.543 |

Volatile solvents

The anhydrous cosmetic composition of the present invention can comprise from about 10% to about 95%, preferably from about 20% to about 80%, more preferably from about 30% to about 70%, by weight of a volatile solvent suitable for topical application. The solvent being a major constituent of a composition which as a whole is required to be anhydrous is preferably anhydrous.

"Volatile solvent" as used herein, refers to those materials that have measurable vapor pressure under ambient conditions. The volatile solvent can comprise a volatile silicone liquid. The concentration of the volatile silicone ranges from about 10% to about 90%, more preferably from about 15% to about 65%, even more preferably from about 30% to about 60%, by weight of the cosmetic stick composition. The volatile silicone may be a cyclic, linear or branched chain silicone having the requisite volatility as defined herein. Non-limiting examples of suitable volatile silicones are described in Todd et al., "Volatile Silicone Fluids for Cosmetics", Cosmetics and Toiletries, 91:27-32 (1976), which descriptions are incorporated herein by reference. Preferred among these volatile silicones are the cyclic silicones having from about 3 to about 7, more preferably from about 5 to about 6, silicon atoms. Most preferably are those which conform to the formula:

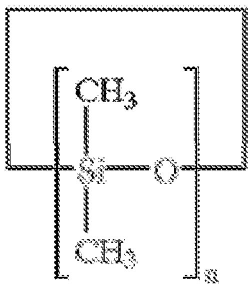

wherein n is from about 3 to about 7, preferably from about 5 to about 6, most preferably 5. These volatile cyclic silicones generally have a viscosity value of less than about 10 centistokes. All viscosity values described herein are measured or determined under ambient conditions, unless otherwise specified. Examples of suitable volatile silicones for use herein include Cyclomethicone D-5 (commercially available from G. E. Silicones); Dow Corning 344, and Dow Corning 345 (commercially available from Dow Corning Corp.); and GE 7207, GE 7158 and Silicone Fluids SF-1202 and SF-1173 (available from General Electric Co.).

In addition to volatile silicone liquids the volatile solvent can also comprise volatile, nonpolar hydrocarbon liquids. In this context, the term "nonpolar" means that these volatile hydrocarbon liquids have a solubility parameter of less than about 7.5 $(cal/cm^3)^{0,5}$, most typically about 5.0 $(cal/cm^3)^{0.5}$ to less than about 7.5 $(cal/cm^3)^{0.5}$. These volatile,

nonpolar hydrocarbon liquids preferably contain only hydrogen and carbon and therefore preferably contain no functional groups. Solubility parameters as described above are determined by methods well known in the chemical arts for establishing the relative polar character of a solvent or other material. A description of solubility parameters and means for determining them are described by C. D. Vaughan, "Solubility Effects in Product, Package, Penetration and Preservation" 103 Cosmetics and Toiletries 47-69, October 1988; and C. D. Vaughan, "Using Solubility Parameters in Cosmetics Formulation", 36 J. Soc. Cosmetic Chemists 319-333, September/October, 1988, which descriptions are incorporated herein by reference.

The nonpolar, volatile hydrocarbon liquid as a liquid carrier for use in the composition of the present invention is preferably a liquid paraffin and/or isoparaffin having the requisite volatility and nonpolar character. The nonpolar, volatile hydrocarbon liquids can have a cyclic, branched and/or chain configuration, and can be saturated or unsaturated, preferably saturated.

Preferred volatile, nonpolar hydrocarbon liquids are branched chain hydrocarbons at a concentration of from about 1% to about 40%, more preferably from about 1% to about 20%, by weight of the composition, and having from about 6 to about 40 carbon atoms, preferably from about 6 to about 20 carbon atoms. These preferred hydrocarbon liquids will most typically be formulated as a combination of two or more of the above-described branched chain hydrocarbons, wherein the combination of two or more hydrocarbons have different molecular weights, number of carbon atoms, and/or chain configurations. Specific nonlimiting examples of such combinations include the isoparaffins available from Exxon Chemical Company, Baytown, Tex. U.S.A, sold as Isopar M (C13-C14 Isoparaffin), Isopar C (C7-C8 Isoparaffin), Isopar E (C8-C9 Isoparaffin), Isopar G (C10-11 Isoparaffin), Isopar L (C11-C13 Isoparaffin), Isopar H (C11-C12 Isoparaffin), and combinations thereof. Other nonlimiting examples of suitable branched chain hydrocarbons include Permethyl 99A (C12, isododecane), Permethyl 101A (C16, isohexadecane), Permethyl 102A (C20, isoeicosane), and combinations thereof. The Permethyl series are available from Presperse, Inc., South Plainfield, N.J., U.S.A. Other nonlimiting examples of suitable branched chain hydrocarbons include petroleum distallates such as those available from Phillips Chemical as Soltrol 130, Soltrol 150, Soltrol 170, and those available from Shell as Shell Sol-70, -71, and -2033.

Still other suitable isoparaffins include C9-C11 Isoparaffin, C9-C13 Isoparaffin, C9-C14 Isoparaffin, C10-C13 Isoparaffin, C12-C14 Isoparaffin, C13-C16 Isoparaffin, C14-C18 Isoparaffin, and hydrogenated polyisobutene available from Amoco as the Panalane Series and from Fanning Corporation as the Fancor P series.

Nonlimiting examples of other volatile, nonpolar hydrocarbon liquids suitable for use in the cosmetic stick compositions include paraffins such as dodecane, octane, decane and combinations thereof, and the Norpar series of paraffins available from Exxon Chemical Company such as Norpar-12, -13, and -15 and the Neosolve series of paraffins available from Shell. Yet another example includes C11-C15 alkanes/cycloalkanes, such as those available from Exxon as Exxsol D80.

## Non-volatile solvent

The anhydrous cosmetic composition compositions of the present invention also can optionally comprise a non-volatile solvent. These non-volatile solvents may be either non-volatile organic fluids or non-volatile silicone fluids.

### 1. Non-Volatile Organic Fluids

The non-volatile organic fluid can be present at concentrations ranging from about 1%, from about 2% but no more than about 20% or no more than about 15%, by weight of the composition. Non-limiting examples of nonvolatile organic fluids include, but are not limited to, mineral oil, PPG-14 butyl ether, isopropyl myristate, petrolatum, butyl stearate, cetyl octanoate, butyl myristate, myristyl myristate, C12-15 alkylbenzoate (e.g., Finsolv.(TM)), dipropylene glycol dibenzoate, PPG-15 stearyl ether benzoate and blends thereof (e.g. Finsolv TPP), neopentyl glycol diheptanoate (e.g. Lexfeel 7 supplied by Inolex), octyldodecanol, isostearyl isostearate, octododecyl benzoate, isostearyl lactate, isostearyl palmitate, isononyl/isononoate, isoeicosane, octyldodecyl neopentanate, hydrogenated polyisobutane, and isobutyl stearate. Many such other carrier liquids are disclosed in U.S. Pat. No. 6,013,248 (Luebbe et al.) and U.S. Pat. No. 5,968,489 (Swaile et al).

### 2. Nonvolatile Silicone Fluids

The non-volatile silicone fluid may be a liquid at or below human skin temperature, or otherwise in liquid form within the anhydrous cosmetic composition during or shortly after topical application: The concentration of the non-volatile silicone may be from about 1%, from about 2% but no more than about 15% or no more than about 10%, by weight of the composition. Non-volatile silicone fluids of the present invention may include those that conform to the formula:

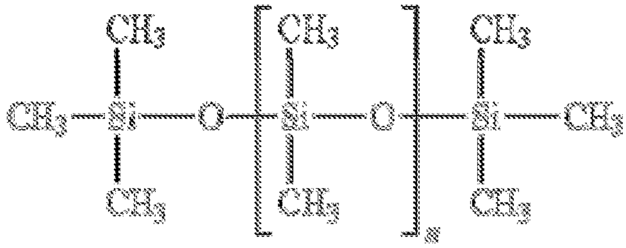

wherein n is greater than or equal to 1. These linear silicone materials may generally have viscosity values of from about 5 centistokes or from about 10 centistokes to about 100,000 centistokes, or to about 500 centistokes or to about 200 centistokes or to about 50 centistokes, as measured under ambient conditions. Specific non limiting examples of suitable nonvolatile silicone fluids include Dow Corning 200, hexamethyldisiloxane, Dow Corning 225, Dow Corning 1732, Dow Corning 5732, Dow Corning 5750 (available from Dow Corning Corp.); and SF-96, SF-1066 and SF 18(350) Silicone Fluids (available from G.E. Silicones).

Low surface tension non-volatile silicone fluids may be also be used. Such solvents may be selected from the group consisting of dimethicones, dimethicone copolyols, phenyl trimethicones, alkyl dimethicones, alkyl methicones, and mixtures thereof. Low surface tension non-volatile solvents are also described in U.S. Pat. No. 6,835,373 (Kolodzik et al.).

In general it is preferred that in the anhydrous cosmetic composition of the present invention the weight ratio of volatile to non-volatile solvent is at least 1:1 and more preferably at least 2:1. Using these preferred ratios provides the additional benefit of reducing the risk of staining the garments which come in contact with the skin treated with the compositions of the invention.

## Thickening agents

The anhydrous cosmetic composition compositions of the present invention also can comprise thickening agents to help provide the composition with the desired viscosity, rheology, texture and/or product hardness, or to otherwise help suspend any dispersed solids or liquids within the composition. The term "thickening agent" may include any material known or otherwise effective in providing suspending, gelling, viscosifying, solidifying or thickening properties to the composition or which otherwise provide structure to the final product form. These thickening agents may include gelling agents, polymeric or nonpolymeric agents, inorganic thickening agents, or viscosifying agents. The thickening agents may include organic solids, silicone solids, crystalline or other gellants, inorganic particulates such as clays or silicas, or combinations thereof.

Preferred thickening agents are Gellants, for example triglyceride gellants.

The concentration and type of the thickening agent selected for use in the anhydrous cosmetic composition of the present invention will vary depending upon the desired product form, viscosity, and hardness. The thickening agents suitable for use herein, may have a concentration range from 2% to 45% by weight of the composition.

Non-limiting examples of suitable gelling agents of the present invention include fatty acid gellants, salts of fatty acids, hydroxyl acids, hydroxyl acid gellants, esters and amides of fatty acid or hydroxyl fatty acid gellants, cholesterolic materials, dibenzylidene alditols, lanolinolic materials, fatty alcohols, triglycerides, sucrose esters such as SEFA behenate, inorganic materials such as clays or silicas, other amide or polyamide gellants, and mixtures thereof. Concentrations of all such gelling agents may be from at least about 0.1%, at least about 1%, or at least about 5% and no more than about 25%, no more than about 15%, or no more than about 10%, by weight of the composition.

Suitable gelling agents include fatty acid gellants such as fatty acid and hydroxyl or alpha hydroxyl fatty acids, having from about 10 to about 40 carbon atoms, and ester and amides of such gelling agents. Non-limiting examples of such gelling agents include, but are not limited to, 12-hydroxystearic acid, 12-hydroxylauric acid, 16-hydroxyhexadecanoic acid, behenic acid, eurcic acid, stearic acid, caprylic acid, lauric acid, isostearic acid, and combinations thereof. Preferred gelling agents are 12-hydroxystearic acid, esters of 12-hydroxystearic acid, amides of 12-hydroxystearic acid and combinations thereof.

Other suitable gelling agents include amide gellants such as disubstituted or branched monoamide gellants, monsubstituted or branched diamide gellants, triamide gellants, and combinations thereof, including n-acyl amino acid derivatives such as n-acyl amino acid amides, n-acyl amino acid esters prepared from glutamic acid, lysine, glutamine, aspartic acid, and combinations thereof. Other suitable amide gelling agents are described in U.S. Pat. No. 5,429,816, issued Jul. 4, 1995, and U.S. Pat. No. 5,840,287, filed Dec. 20, 1996.

Still other examples of suitable gelling agents include fatty alcohols having at least about 8 carbon atoms, at least about 12 carbon atoms but no more than about 40 carbon atoms, no more than about 30 carbon atoms, or no more than about 18 carbon atoms. For example, fatty alcohols include but are not limited to cetyl alcohol, myristyl alcohol,

stearyl alcohol and combinations thereof.

Non limiting examples of suitable tryiglyceride gellants include tristearin, hydrogenated vegetable oil, trihydroxysterin (Thixcin(R) R, available from Rheox, Inc.), rape seed oil, castor wax, fish oils, tripalmitin, Syncrowax(R) HRC and Syncrowax(R) HGL-C (Syncrowax(R) available from Croda, Inc.).

Other suitable thickening agents include waxes or wax-like materials having a melt point of above 65° C., more typically from about 65° C. to about 130° C., examples of which include, but are not limited to, waxes such as beeswax, carnauba, bayberry, candelilla, montan, ozokerite, ceresin, hydrogenated castor oil (castor wax), synthetic waxes and microcrystalline waxes. Castor wax is preferred within this group. Other high melting point waxes are described in U.S. Pat. No. 4,049,792, Elsnau, issued Sep. 20, 1977.

Further thickening agents for use in the anhydrous cosmetic composition of the present invention may include inorganic particulate thickening agents such as clays and colloidal pyrogenic silica pigments. For example, colloidal pyrogenic silica pigments such as Cab-O-Sil(R), a submicroscopic particulated pyrogenic silica may be used. Other known or otherwise effective inorganic particulate thickening agents that are commonly used in the art can also be used in the solid antiperspirant compositions of the present invention. Concentrations of particulate thickening agents may range, for example, from at least about 0.1%, at least about 1%, at least about 5% but no more than about 35%, no more than about 15%, no more than about 10% or no more than about 8%, by weight of the composition.

Suitable clay thickening agents include montmorillonite clays, examples of which include bentonites, hectorites, and colloidal magnesium aluminum silicates. These and other suitable clays may be hydrophobically treated, and when so treated will generally be used in combination with a clay activator. Non-limiting examples of suitable clay activators include propylene carbonate, ethanol, and combinations thereof. When clay activators are present, the amount of clay activator will typically range from at least about 40%, at least about 25%, at least about 15% but no more than about 75%, no more than about 60%, or no more than about 50%, by weight of the clay.

## Glycols

The anhydrous cosmetic composition of the present invention preferably comprise a low amount of propylene and dipropylene glycol. Preferably less than 1% wt. more preferably less than 0.1% wt. Even more preferably the anhydrous cosmetic composition of the present invention are free of propylene and dipropylene glycol. These glycols in this context may have a detrimental effect on skin irritation and also contribute to an anticipated release of the complexed or encapsulated compounds contained herein.

## Prebiotics

The anhydrous cosmetic composition of the present invention may comprise prebiotics. Any suitable prebiotic may be included. Some suitable examples of prebiotics include one or more of a carbohydrate, carbohydrate monomer, carbohydrate oligomer, or carbohydrate polymer. Other suitable exemplary prebiotics include inulin, lactose, lactulose, raffinose, stachyose, fructooligossacharides, glucooligosaccharides, lactoferrin, mannan oligosaccharides, glucan oligosaccharides, isomaltooligosaccharides, lactosucrose, polydextrose, soybean oligosaccharides, xylooligosaccharides, paratinose oligosaccharides, transgalactosylated oligosaccharides, transgalactosylate disaccharides, gentiooligosaccharides, pecticoligosaccharides palatinose polycondensates, difructose anhydride III, sorbitol, maltitol, lactitol, polyols, polydextrose, reduced paratinose, cellulose, β-glucose, β-galactose, β-fructose, verbascose, galactinol, β-glucan, guar gum, pectin, sodium alginate, and lambda carrageenan and mixtures thereof. Additional suitable examples include human milk oligosaccharides as disclosed in US2013281948 A1, for example lactose, 2'-fucosyllactose, 3'-fucosyllactose, difucosyllactose, lacto-N-tetraose (type 1), lacto-N-neo-tetraose (type 2), lacto-N-fucopentaoses I, II, III, IV and V, lacto-N-fucohexaose I, lacto-N-hexaose, lacto-N-neohexaose, fucosyllacto-Nhexaose I and IV, fucosyllacto-N-neohexaose, lacto-N-difuco-hexaoses I and II, lacto-Noctaoses, sialya2-3lactose, sialya2-6lactose, sialyl-lacto-N-tetraose a, b and c, and disialyl-lacto-N-tetraose, and mixtures thereof. Still other suitable examples prebiotics include fucosea(1®2)galalactose b as a disaccharide unit, 2'-fucosyllactose, 3'-fucosyllactose, lacto-N-difuco-tetraose, lacto-N-difuco-hexose I, lacto- N-difuco-hexose II, lacto-N-fucopentaose I, lacto-N-fucopentaose II, lacto-N-fucopentaose III, lacto-N-fucopentaose V, and mixtures thereof.

In some forms of the present invention, the prebiotic may comprise carob bean, citrus pectin, rice bran, locust bean, fructooligosaccharide, oligofructose, galactooligosaccharide, citrus pulp, annanoligosaccharides, arabinogalactan, lactosucrose, glucomannan, polydextrose, apple pomace, tomato pomace, carrot pomace, cassia gum, gum karaya, gum talha, gum arabic, and combinations thereof.

## Optional Ingredients

The anhydrous cosmetic composition of the present invention may further comprise any optional material that

is known for use in cosmetic compositions or other personal care products, or which are otherwise suitable for topical application to human skin. Non limiting examples of such other optional materials include dyes or colorants, emulsifiers, distributing agents, residue masking agents, inert fillers, preservatives, surfactants, processing aides such as viscosity modifiers, wash-off aids, and so forth. Other suitable optional materials include other solid gellants or waxes in addition to and other than the gellants described herein. Examples of such optional materials are described in U.S. Pat. No. 4,049,792 (Elsnau); U.S. Pat. No. 5,019,375 (Tanner et al.); and U.S. Pat. No. 5,965,113 (Guskey), which descriptions are incorporated herein by reference.

It is especially preferred in the case of application to the intimate area for the composition of the present invention to be free of ingredients such as water that can promote growth of bacteria. It is also preferred for the compositions of the present invention to contain enough bacteria growth inhibiting ingredients to prevent bacteria from growing on the stick after application and reapplication to the intimate area (to prevent re-infection, in the case of bacterial infections such as bacterial vaginosis) typically at a level greater than 0.01%wt. The most preferred actives are Zinc Oxide or Hexamidine. Alternately, any acidic ingredient that lowers pH may be used to prevent or retard bacteria growth on the stick surface.

Product forms: solid stick

The anhydrous cosmetic composition of the present invention can be for example in the form of a solid composition, e.g., as a solid stick. Cosmetic compositions in the form of sold sticks are known in the art and commonly used especially in the field of deodorants. Typically the solid stick has a waxy consistency and is commercialized within an applicator which facilitates application onto the skin. Applicators for topical compositions in stick form are known in the art and commonly marketed. When the stick is rubbed onto the skin a thin layer of composition is applied onto the skin due to the mechanical action and or the temperature of the body which partially melts the solid stick material on its surface. Solid stick product forms can hold an advantage over other product forms (such as, for example, lotions, creams and gels) because no drying or waiting is required before donning one's undergarments after rubbing the composition onto skin/hair in the intimate area. Solid stick product forms can also hold an advantage over sprays and other non-touch applications in that the user can more easily and accurately control application of the composition to skin the intimate area.

The solid stick compositions preferably comprise 5-45% wt. of a thickening agent which is a gellant and have a product hardness of at least about 600 gram.force.

In some forms of the present invention, the composition of the stick may be such that the stick is opaque, e.g. white. As applied, the stick may leave an opaque residue on the skin, e.g. white. The visible residue can be helpful for the user to see any areas that may have been missed. In some forms, as applied, the stick may leave a translucent residue on the skin. For example, for those forms where an opaque residue is desired, particles sizes of, for example, cosmetic actives may be chosen such that an opaque residue is achieved.

For those forms where a translucent residue is desired, the particle sizes of, for example, cosmetic actives may be chosen such that a translucent residue is achieved. For example, for those formulations utilizing zinc oxide, the particle size may be in the range of less than 100 nm - nano zinc oxide.

In addition, the composition of the invention can be formulated to comprise elements that protect skin against insults from body fluids or mechanical damage by providing a substantive protective film on the skin including petrolatum, dimethicone and solid wax or triglyceride gellants. The composition can comprise hydrophobic panthenol derivatives that soften intimate hair to make it easier to shave, reduces shave bumps and reduces intimate itching as hair re-grows. For women facing urine leakage, additional protection can be added via Zinc Oxide that further helps with urine damaged skin. The composition can also be formulated by including non-volatile emollients that help to improve glide on application and to reduce residue impression, but kept at a level low enough as to prevent leaving visible greasy stains in intimate undergarments.

In some forms, the substantive protective film may comprise about 500 micrograms / $cm^2$ to about 5000 microgram/$cm^2$ of deposition of composition to intimate skin where applied. In some forms of the present invention, in addition to the foregoing range, the composition deposition levels may be from about 750 micrograms/$cm^2$ to about 4000 microgram/$cm^2$ or from about 1000 micrograms/$cm^2$ to about 3500 micrograms/$cm^2$, or any values within these ranges or any ranges created thereby.

Examples

The following non-limiting examples illustrate specific embodiments of the cosmetic stick compositions of the present invention. Each is formulated by combining the solid thickening agents (gellants) and liquid carriers in a vessel equipped with a heat source. The combined solids and liquids are heated to a temperature ranging from 85°C to 96°C and agitated to dissolve the solid thickening agents until the mixture forms a homogeneous clear to slightly cloudy

solution, at which point any solid cosmetic active is added to and dispersed throughout the heated solution while maintaining mixing. The complexed or encapsulated compounds are then added to the melted mixture. If using a cyclodextrin complex it is preferable to use a cyclodextrin complex with a degree of complexing greater than about 75% to prevent perfume from flashing or evaporating in the heating stage of the making process and to prevent compounds from the complex from blending in with the free perfume added separately. If needed, the mixture may then be milled to eliminate solid particles or clumps and render the composition smooth and free of large particles. It is especially preferred to use a spray dried cyclodextrin complex because the particles are free of hard glassy edges that will scratch the skin during use. Other methods of producing the complex (for example via extrusion, or via solvent crystallization) create hard glassy particles that scratch the agents (gellants) and liquid carriers in a vessel equipped with a heat source. The combined solids and liquids are heated to a temperature ranging from 85°C to 96°C and agitated to dissolve the solid thickening agents until the mixture forms a homogeneous clear to slightly cloudy solution, at which point any solid cosmetic active is added to and dispersed throughout the heated solution while maintaining mixing. The complexed or encapsulated compounds are then added to the melted mixture. If using a cyclodextrin complex it is preferable to use a cyclodextrin complex with a degree of complexing greater than about 75% to prevent perfume from flashing or evaporating in the heating stage of the making process and to prevent compounds from the complex from blending in with the free perfume added separately. If needed, the mixture may then be milled to eliminate solid particles or clumps and render the composition smooth and free of large particles. It is especially preferred to use a spray dried cyclodextrin complex because the particles are free of hard glassy edges that will scratch the skin during use. Other methods of producing the complex (for example via extrusion, or via solvent crystallization) create hard glassy particles that scratch the skin. Finally, an optional perfume may be added at this stage, just prior to or after cooling. The resulting heated combination is then circulated through a scraped wall heat exchanger and cooled to 62°C before filling the cooled mixture into plastic dispensing canisters and allowed to cool and solidify within the canisters over a 20 minute period (cooling rate of 2°C/min) through a forced air cooling tunnel having an air temperature of 21°C. The exemplified compositions are then placed in a constant temperature room maintained at 25°C for a period of one week (7 days) after which they are evaluated for hardness according to the method described herein. Each of the exemplified compositions is applied topically to the appropriate area of the skin, in accordance with the methods of use described herein.

All exemplified amounts are weight percentages based upon the total weight of the cosmetic stick composition, unless otherwise specified.

| TABLE 2: COMPONENT MIXTURE | |
|---|---|
| INGREDIENT | AMOUNT (wt%) |
| Intreleven Aldehyde | 2 |
| Florhydral | 20 |
| Floral Super | 10 |
| Scentenal | 5 |
| Cymal | 25 |
| Floralozone | 10 |
| Adoxal | 1 |
| Methyl NonylAcetaldehyde | 1 |
| Melonal | 1 |
| o-anisaldehyde | 25 |

Table 3 - Anhydrous Solid Cosmetic Sticks and Creams

| Ingredient | Example 1 | Example 2 | Example 3 | Example 4 | Example 5 |
|---|---|---|---|---|---|
| | AI Stick | Shaver's Stick | AI Cream | AI + PMC | Unscented Cream |
| Ozokerite Wax | 16.00 | 17.00 | 0.00 | 0.00 | 0.00 |
| Cyclopentasiloxane | 46.81 | 46.81 | 66.312 | 66.312 | 66.812 |

(continued)

| Ingredient | Example 1 | Example 2 | Example 3 | Example 4 | Example 5 |
|---|---|---|---|---|---|
| | AI Stick | Shaver's Stick | AI Cream | AI + PMC | Unscented Cream |
| Tribehenin (Syncrowax HR-C)[1] | 0.00 | 0.00 | 7.75 | 7.75 | 7.75 |
| C18-36 Acid Triglyceride (Syncrowax HGLC)[2] | 0.00 | 0.00 | 1.938 | 1.938 | 1.938 |
| Perfume | 0.00 | 0.00 | 0.50 | 0.50 | 0.00 |
| Petrolatum | 4.00 | 5.00 | 5.00 | 5.00 | 5.00 |
| Spray Dried Beta Cyclodextrin Feminine Odor Control Perfume Complex of table 1 (95% Degree Of Complexing) | 5.00 | 5.00 | 5.00 | 0.00 | 5.00 |
| Zinc Oxide | 5.00 | 0.00 | 0.50 | 0.50 | 0.50 |
| Dimethicone 50 cst | 3.00 | 3.00 | 5.00 | 5.00 | 5.00 |
| C12-C15 Alkyl Benzoate | 10.00 | 10.00 | 0.00 | 0.00 | 0.00 |
| PPG 14 Butyl Ether (Fluid AP) | 10.00 | 10.00 | 5.00 | 5.00 | 5.00 |
| Behenyl Alcohol | 0.19 | 0.19 | 0.00 | 0.00 | 0.00 |
| Panthenyl Triacetate | 0.00 | 3.00 | 3.00 | 3.00 | 3.00 |
| Spray Dried Polyacrylate Microcapsules Containing Feminine Odor Control Perfume of Table 2 | 0.00 | 0.00 | 0.00 | 5.00 | 0.00 |
| Added Water | 0.00 | 0.00 | 0.00 | 0.00 | 0.00 |
| Antiperspirant Active (e.g., Aluminum Zirconium Tetrachlorohydrex Gly) | 0.00 | 0.00 | 0.00 | 0.00 | 0.00 |
| Propylene Glycol / Dipropylene Glycol | 0.00 (1.00 <) (0.10<) | 0.00 (1.00 <) (0.10<) | 0.00 | 0.00 | 0.00 |
| | | | | | |
| Totals | 100.00 | 100.00 | 100.00 | 100.00 | 100.00 |
| | | | | | |
| Product hardness (gram·force) | 1102 | 920 | 220 | 205 | 215 |
| 1. Croda, Inc., New York, New York, USA<br>2. Croda, Inc., New York, New York, USA | | | | | |

Table 4 - Anhydrous Solid Cosmetic Sticks

| Ingredient | Example 6 | Example 7 |
|---|---|---|
| Cyclomethicone | 45.99 | 48.99 |
| Fluid AP | 5.00 | 5.00 |
| Dimethicone 50 cst | 5.00 | 5.00 |
| Mineral Oil | 8.00 | 8.00 |
| Petrolatum | 5.00 | 5.00 |

(continued)

| Ingredient | Example 6 | Example 7 |
|---|---|---|
| Stearyl Alcohol | 16.00 | 16.00 |
| Hydrogenated Castor Oil | 3.56 | 3.56 |
| Behenyl Alcohol | 0.20 | 0.20 |
| Performathox 450 Ethoxylate | 1.00 | 1.00 |
| Talc | 5.50 | 5.50 |
| Glycerin | 0.50 | 0.50 |
| Polyacrylate Microcapsules Containing Feminine Odor Control Perfume of Table 2 | 3.00 | 0.00 |
| Fragrance | 1.25 | 1.25 |
| Antiperspirant Active | 0.00 | 0.00 |

The above compositions are particularly effective when applied to the intimate area prior to application of an adult incontinence article (liner, pad or pants intended to capture urine leakage through the day). Because these sticks can be applied at much higher levels to the skin than is possible via previous methods of delivering skin actives via lotioned embibed absorbent articles, the effect is more pronounced in keeping wetness away from the skin and giving users greater skin comfort and odor protection throughout the day than was previously possible.

Alternately, this new form is particularly useful when applied to baby's skin after each diaper change to prevent diaper rash.

The present disclosure provides methods for grooming intimate area hair. Exemplary methods include the steps of: (a) cutting or removing hair from skin in the intimate area by at least one of the following: shaving, trimming, laser hair removal, epilation, waxing, chemical methods, and the like; (b) thereafter prepping the intimate area skin for application of a cosmetic product provided herein; (c) and rubbing an anhydrous cosmetic composition to the intimate area skin, wherein the anhydrous cosmetic composition is in the form of a solid stick. Prepping step (b) can include, for example, removing shaven hair, rinsing and/or washing the skin that has been shaved, and/or drying the skin that has been shaved. Step (a) can employ a razor that comprises a lubrication strip and/or a water-soluble cosmetic composition that is used in the place of a separately packaged shaving composition. Step (a) can also employ a trimmer, scissors or other device suitable for cutting human hair. These processes for step (a) focus on cutting hair in the intimate area either at or near skin level or some distance beyond the skin. These processes can cause irritation to the skin in the intimate area. Other suitable process for step (a) are contemplated. For example, laser hair removal, epilation, waxing, chemical methods, the like, or combinations thereof. In general these processes can similarly cause skin irritation just as the cutting processes described heretofore. Additionally, in some forms, while some may choose to skip step (b), the prepping of the skin, efficacy of the composition may negatively impacted if for example, shaven hair is left on the intimate skin and/or the intimate skin is wet.

The present disclosure also provides methods for managing incontinence. Exemplary methods include the steps of: (a) rubbing an anhydrous cosmetic composition onto skin and/or pubic hair in the intimate area, wherein the anhydrous cosmetic composition is in the form of a solid stick; (b) applying an incontinence absorbent article against the intimate skin area comprising the anhydrous cosmetic composition, wherein the incontinence absorbent article is a liner, a pad, or a pant.

Absorbent articles are generally in the form of a pad/liner or a pant (brief). While the absorbent articles useful for managing incontinence may take on various design configurations, they typically employ a liquid permeable topsheet, a liquid impermeable backsheet, and an absorbent structure disposed therebetween. Fig. 1 illustrates an example of an absorbent article in the form of a pant 20. The absorbent article has an outer surface 22, an inner surface 24 opposed to the outer surface 22, a front waist region 26, a rear waist region 28, a crotch region 30, and seams 32 which join the front waist region 26 and the rear waist region 28 to form two leg openings 34 and a waist opening 36. The seams 32 may be permanent or refastenable.

Pant 20 may comprise an absorbent chassis 38 to cover a crotch region of a wearer and a belt 40 extending transversely about the waist opening 36. The pant 20 may also optionally comprise an outer cover layer 42 to cover the chassis 38. The belt 40 may define the waist opening 36 in the pant 20. The belt 40, the chassis 38, and/or the outer cover layer 42 may jointly define the leg openings 34. In one embodiment, the pant 20 may have a patch sheet 44 printed with a graphic 46 thereon, which may be disposed in the front waist region 26, the rear waist region 28, or any other suitable portion of the pant 20. The belt 40 may be formed from a front belt 84 in the front waist region 26 and a rear

belt 86 in the rear waist region 28. The front belt 84 may form a front waist edge 35 in the front waist region 26 and the rear belt 86 may form a rear waist edge 37 in the rear waist region 28. The front and rear waist edges 35 and 37 may be laterally opposed about the lateral central axis. The belt 40 may form a portion of an outer surface 22 or an inner surface 24 of the pant 20. In other embodiments, the belt 40, or portions thereof, may be disposed intermediate other layers of the chassis 38, such as a topsheet and a backsheet, for example.

Pant 20 may comprise front and rear belts 84 and 86 intended to encircle at least a portion of the waist of the wearer. The front and rear belts 84 and 86 together form at least a portion of, or all of, the belt 40 when joined. The front and rear belts 84 and 86 may be connected by the chassis 38 forming the crotch region 30 of the pant 20. The front and rear belts 84 and 86 may each be formed from a first belt layer 82 possibly forming a portion of the outer surface 22 of the pant 20 and a second belt layer 83 possibly forming a portion of the inner surface 24 of the pant 20. The first and second belt layers 82 and 83 may be comprised of any known materials. Various suitable materials may comprise films, plastic films, apertured plastic films, woven or nonwoven webs of natural materials (e.g., wood or cotton fibers), synthetic fibers (e.g., polyolefins, polyamides, polyester, polyethylene, or polypropylene fibers), or a combination of natural and/or synthetic fibers, stretchable nonwovens, or coated woven or nonwoven webs. The belt 40 may comprise an inner hydrophobic, nonwoven material and an outer hydrophobic, nonwoven material. The front and rear belts 84 and 86 may also comprise a plurality of elastic elements 85 disposed at least partially between the first and second belt layers 82 and 83 thereof and attached to at least one of the first and second belt layers 82 and 83 using adhesives or bonding, for example. The elastic elements 85 may comprise one or more elastic strands, elastic materials, elastomeric films, elastomeric ribbons, elastomeric nonwovens, elastomeric filaments, elastomeric adhesives, elastomeric foams, scrims, or combinations thereof

The chassis 38 of the pant 20 may comprise a portion of the outer surface 22, a backsheet 60, a portion of the inner surface 24, a topsheet 58, and an absorbent core 62 disposed between at least a portion of the topsheet 58 and the backsheet 60. In addition, the chassis 38 may comprise elasticized barrier leg cuffs 64 disposed at or adjacent the side edges 48 of the chassis 38. The barrier leg cuffs 64 may provide improved containment of liquids and other body exudates or wastes in the crotch region 30 and may comprise a single layer of material which may be folded to form a barrier leg cuff having two layers. The barrier leg cuffs 64 may extend from the side of the chassis 38 at or adjacent the longitudinal side edge 48 toward the longitudinal central axis LI. The barrier leg cuffs 64 may be folded along the folding lines 66 back toward the longitudinal side edges 48. The front and rear belts 84 and 86 may overlap at least a portion of the chassis 38 and one or both of the front and rear belts 84 and 86 may be disposed on the outer surface 22 of the chassis 38, on the inner surface 24 of the chassis 38, or disposed intermediate various portions of the chassis 38.

In one embodiment, a portion of, or the whole of, the chassis 38 may be made extensible to a degree greater than the inherent extensibility of the material or materials from which the chassis 38 is made, e.g., the backsheet 60. The additional extensibility may be desirable in order to allow the chassis 38 to conform to the body of a wearer during movement by the wearer and or to provide adequate body coverage. The additional extensibility may also be desirable, for example, in order to allow the user of a pant including the chassis 38 having a particular size before extension to extend the front waist region 26, the rear waist region 28, or both of the waist regions of the chassis 38 to provide additional body coverage for wearers of differing size, i.e., to tailor the pant to the individual wearer. Such extension of the waist region or regions may give the chassis 38 a generally hourglass shape, so long as the crotch region 30 is extended to a relatively lesser degree than the waist region or regions, and may impart a tailored appearance to the pant 20 when it is donned or worn. In addition, the additional extensibility may be desirable in order to minimize the cost of the pant 20. For example, an amount of material that would otherwise be sufficient only to make a relatively smaller pant lacking this extensibility may be used to make an article capable of being extended to adequately cover a wearer that is larger than the unextended smaller pant would fit.

A portion of the chassis 38, for example, a portion of the chassis 38 in one or both of the waist regions 26 and 28 may be made laterally extensible to a maximum extensibility greater than a maximum extensibility of another portion of the chassis 38 in the crotch region 30 such that a lateral extension of each of the portions to its maximum extensibility imparts an hourglass shape to the chassis 38. In one embodiment, the portion of the chassis 38 underlying, overlying, and/or immediately adjacent one or both of the front and rear extensible belts 84 and 86 may be made laterally extensible to a maximum extensibility greater than a maximum extensibility of another portion of the chassis 38, for example the crotch region 30, such that a lateral extension of each of the portions to its maximum extensibility facilitates application of the pant 20 onto the body of a wearer by enabling the waist regions 26 and 28 to be extended to fit over the wearer's hips and in addition, opening and orienting the leg openings enabling the wearer to place the legs through the openings more effectively.

In one particular form, the liquid pervious topsheet 58 may be positioned adjacent the body-facing surface of the absorbent core 62 and may be joined thereto and/or to the backsheet 60 by any attachment means known to those of skill in the art. The liquid impervious backsheet 60 may generally be that portion of the pant 20 positioned adjacent the garment-facing surface of the absorbent core 62 and may prevent, or at least inhibit, the bodily exudates and wastes absorbed and contained in the absorbent core 62 from soiling garments that may contact the outer surface 22 of the

pant 20.

The topsheet 58, the backsheet 60, and the absorbent core 62 may be manufactured of any known materials. Suitable topsheet materials may comprise porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Suitable backsheet materials may include breathable materials that permit vapors to escape from the pant 20 while still preventing, or at least inhibiting, bodily exudates or wastes from passing through the backsheet 60. Such materials may include nonwoven materials, woven materials, films, and/or laminates comprising a combination of one or more of these materials. In one embodiment, the backsheet 60 may be a film and nonwoven laminate, wherein the nonwoven of the laminate forms the outer cover layer 42.

A suitable absorbent core 62 for use in the pant 20 may comprise any absorbent material which is generally compressible, conformable, non-irritating to the wearer's skin, and capable of absorbing and retaining liquids such as urine and other certain body exudates. In addition, the configuration and construction of the absorbent core 62 may also be varied (e.g., the absorbent core(s) or other absorbent structure(s) may have varying caliper zones, hydrophilic gradient(s), a superabsorbent gradient(s), or lower average density and lower average basis weight acquisition zones; or may comprise one or more layers or structures). In some embodiments, the absorbent core 62 may comprise a fluid acquisition component, a fluid distribution component, and/or a fluid storage component. An example of a suitable absorbent core having a fluid acquisition component, a fluid distribution component, and a fluid storage component is described in U.S. Pat. No. 6,590,136.

In one particular form, the outer cover layer 42 may be disposed on the outer surface 22 of the pant 20 and may cover the crotch panel 56 of the absorbent chassis 38. The outer cover layer 42 may extend into and cover the front waist panel 52 and the rear waist panel 54 of the chassis 38. The outer cover layer 42 may form a portion of the backsheet 60 and/or the chassis 38. In one embodiment, the outer cover layer 42 may be directly joined to and cover a portion of, or all of, the liquid impervious backsheet 60 of the chassis 38. In various embodiments, the outer cover layer 42 may be disposed between the front and rear belts 84 and 86.

The outer cover layer 42 may comprise a material separate from the first and second belt layers 82 and 83 forming the belts 84 and 86. The outer cover layer 42 may comprise two or more layers of materials of any known materials including the materials used for the first and second belt layers 82 and 83. In one embodiment, the outer cover layer 42 may comprise a single layer of a nonwoven web of synthetic fibers. In various embodiments, the outer cover layer 42 may comprise a single layer of hydrophobic, non-stretchable nonwoven material. In one embodiment, the outer cover layer 42 may comprise a film, a foam, a nonwoven, a woven material, or the like and/or combinations thereof such as a laminate of a film and a nonwoven.

In one embodiment, the belt 40 may be at least partially formed, or fully formed, when the front and rear belts 84 and 86 are permanently or refastenably connecting together to form the seams 32. Any suitable seams may be formed, as known to those of skill in the art. The belt 40 may be ring-like and elastic. The ring-like elastic belt 40 may extend about the waist opening 36 of the pant 20 and act to dynamically create fitment forces and to distribute the forces dynamically generated during wear.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." In all instances in the present application wherein a percentage is mentioned it is intended to indicate a percentage by weight, unless specified otherwise.

## Claims

1. Use of an anhydrous cosmetic composition for neutralizing malodour by topical application in intimate areas, the anhydrous cosmetic composition comprising:
   a volatile solvent, and one or more complexed or encapsulated compounds selected from the group consisting of: melonal, adoxal, trans-2-hexenal, ligustral, floral super, forhydral, 5-methyl-2-thiopene-carboxaldehyde, hydratropic aldehyde, undecanal, 9-undecenal, 10-undecenal, trans-4-decenal, cis-6-nonenal, isocyclocitral, precyclemone b, €-2-(z)-6-nonadienal, undecyl aldehyde, methyl-octyl-acetaldehyde, lauric aldehyde, silvial, vanillin, and floralozone wherein the anhydrous cosmetic composition is in the form of a solid stick comprising a product hardness of at least 600 gram-force (as measured with the method described in the description); and wherein the anhydrous cosmetic composition is devoid of an antiperspirant active.

2. The use of the anhydrous cosmetic composition according to claim 1, wherein said one or more compounds are starch encapsulated.

**3.** The use of the anhydrous cosmetic composition according to any of the preceding claims, wherein said one or more compounds are encapsulated in reservoir type capsules and can be released by a mechanical action breaking the shell of the capsule.

**4.** The use of the anhydrous cosmetic composition according to any of the preceding claims, wherein the one or more compounds are comprised as cyclodextrin complexes.

**5.** The use of the anhydrous cosmetic composition according to any of the preceding claims, further comprising a cosmetic active, wherein the cosmetic active is zinc oxide.

**6.** The use of the anhydrous cosmetic composition according to claims 1-4, further comprising a cosmetic active, wherein the cosmetic active is panthenyl triacetate.

**7.** The use of the anhydrous cosmetic composition according to claims 1-4, and 6 further comprising a cosmetic active selected from the group consisting of zinc oxide and an oil-soluble Vitamin B5 derivative.

**8.** The use of the anhydrous cosmetic composition of any of the preceding claims, wherein the product hardness is from about 800 gram-force to about 1,400 gram-force.

**9.** The use of the anhydrous cosmetic composition of any of the preceding claims, wherein the anhydrous cosmetic composition comprises less than 1 weight percent of propylene glycol and dipropylene glycol.

**10.** A method of treating skin in the intimate area to neutralize malodour, the method comprising the steps of:
rubbing an anhydrous cosmetic composition that is devoid of an antiperspirant active onto skin and/or hair in an intimate skin area to apply the anhydrous cosmetic composition to the same, the anhydrous cosmetic composition comprising:

a product hardness of at least 600 gram-force; and a volatile solvent, and
one or more complexed or encapsulated compounds selected from the group consisting of: melonal, adoxal, trans-2-hexenal, ligustral, floral super, forhydral, 5-methyl-2-thiopene-carboxaldehyde, hydratropic aldehyde, undecanal, 9-undecenal, 10-undecenal, trans-4-decenal, cis-6-nonenal, isocyclocitral, precyclemone b, €-2-(z)-6-nonadienal, undecyl aldehyde, methyl-octyl-acetaldehyde, lauric aldehyde, silvial, vanillin, and floralozone.

**11.** The method of claim 10 further comprising the steps of (i) shaving or trimming hair from skin in the intimate skin area, and (ii) thereafter, prepping the skin in the intimate skin area for application of a cosmetic composition each of which occurs prior to the step of application in claim 10.

**12.** The method of claim 10, further comprising the step of applying an incontinence absorbent article against the skin and/or hair in the intimate skin area comprising the anhydrous cosmetic composition, the incontinence absorbent article comprising a liner, a pad, or a pant.

**13.** The method of claims 10 and 11, wherein the anhydrous cosmetic composition comprises a cosmetic active which is an oil soluble derivative of Vitamin B5.

**14.** The method of claims 10 and 12, wherein the anhydrous cosmetic composition comprises a cosmetic active which is zinc oxide.

**15.** The method according to any of the preceding claims, wherein the anhydrous cosmetic composition provides a substantive protective film upon application to the skin in the intimate area.

**Patentansprüche**

**1.** Verwendung einer wasserfreien Kosmetikzusammensetzung zur Neutralisierung von schlechten Gerüchen durch örtliche Anwendung in Intimbereichen, wobei die wasserfreie Zusammensetzung Folgendes umfasst:
ein flüchtiges Lösungsmittel und eine oder mehrere komplexe oder eingekapselte Verbindungen ausgewählt aus der Gruppe bestehend aus: Melonal, Adoxal, Trans-2-hexanal, Ligustral, Floral super, Forhydral, 5-Methyl-2-thiophen-carboxaldehyd, hydrotrophes Aldehyd, Undecanal, 9-Undecanal, 10-Undecanal, Trans-4-decenal, Cis-6-nonenal, Isocyclocitral, Precyclemon B, €-2-(z)-6-nonadienal, Undecylaldehyd, Methyl-octyl-acetaldehyd, Laurinaldehyd, Silvial, Vanillin und Floralozon, wobei die wasserfreie Kosmetikzusammensetzung in der Form eines festen Stifts vorliegt, umfassend eine Produkthärte von mindestens 600 Gramm-Kraft (gemessen mit dem in der Beschreibung beschriebenen Verfahren); und wobei die wasserfreie Kosmetikzusammensetzung frei von einem schweißhemmenden Wirkstoff ist.

**2.** Verwendung der wasserfreien Kosmetikzusammensetzung nach Anspruch 1, wobei die eine oder die mehreren Verbindungen in Stärke eingekapselt sind.

**3.** Verwendung der wasserfreien Kosmetikzusammensetzung nach einem der vorstehenden Ansprüche, wobei die eine oder die mehreren Verbindungen in Kapseln vom Reservoirtyp eingekapselt sind und durch eine mechanische Wirkung, die die Schale der Kapsel bricht, freigesetzt werden können.

**4.** Verwendung der wasserfreien Kosmetikzusammensetzung nach einem der vorstehenden Ansprüche, wobei die eine oder die mehreren Verbindungen als Cyclodextrinkomplexe umfasst sind.

**5.** Verwendung der wasserfreien Kosmetikzusammensetzung nach einem der vorstehenden Ansprüche, ferner umfassend einen kosmetischen Wirkstoff, wobei der kosmetische Wirkstoff Zinkoxid ist.

**6.** Verwendung der wasserfreien Kosmetikzusammensetzung nach Anspruch 1 bis 4, ferner umfassend einen kosmetischen Wirkstoff, wobei der kosmetische Wirkstoff Panthenyltriacetat ist.

**7.** Verwendung der wasserfreien Kosmetikzusammensetzung nach Anspruch 1 bis 4 und 6, ferner umfassend einen kosmetischen Wirkstoff ausgewählt aus der Gruppe bestehend aus Zinkoxid und einem öllöslichen Vitamin-B5-Derivat.

**8.** Verwendung der wasserfreien Kosmetikzusammensetzung nach einem der vorstehenden Ansprüche, wobei die Produkthärte etwa 800 Gramm-Kraft bis etwa 1 400 Gramm-Kraft beträgt.

**9.** Verwendung der wasserfreien Kosmetikzusammensetzung nach einem der vorstehenden Ansprüche, wobei die wasserfreie Kosmetikzusammensetzung weniger als 1 Gewichtsprozent Propylenglycol und Dipropylenglycol umfasst.

**10.** Verfahren zur Behandlung von Haut in dem Intimbereich zum Neutralisieren von schlechten Gerüchen, wobei das Verfahren die folgenden Schritte umfasst:
Einreiben einer wasserfreien Kosmetikzusammensetzung, die frei von einem schweißhemmenden Wirkstoff ist, in Haut und/oder Haare in einem Intimhautbereich, um die wasserfreie Kosmetikzusammensetzung auf derselben/denselben anzuwenden, wobei die wasserfreie Kosmetikzusammensetzung Folgendes umfasst:

eine Produkthärte von mindestens 600 Gramm-Kraft; und ein flüchtiges Lösungsmittel und
eine oder mehrere komplexe oder eingekapselte Verbindungen, ausgewählt aus der Gruppe bestehend aus: Melonal, Adoxal, Trans-2-hexanal, Ligustral, Floral super, Forhydral, 5-Methyl-2-thiophen-carboxaldehyd, hydrotrophes Aldehyd, Undecanal, 9-Undecanal, 10-Undecanal, Trans-4-decenal, Cis-6-nonenal, Isocyclocitral, Precyclemon B, €-2-(z)-6-nonadienal, Undecylaldehyd, Methyl-octyl-acetaldehyd, Laurinaldehyd, Silvial, Vanillin und Floralozon.

**11.** Verfahren nach Anspruch 10, ferner umfassend die Schritte des (i) Rasierens oder Trimmens von Haaren von Haut in dem Intimhautbereich und (ii) danach des Vorbereitens der Haut in dem Intimhautbereich zur Anwendung einer Kosmetikzusammensetzung, wobei jeder davon vor dem Schritt der Anwendung in Anspruch 10 geschieht.

**12.** Verfahren nach Anspruch 10, ferner umfassend den Schritte des Anwenden eines Inkontinenzabsorptionsartikels auf der Haut und/oder den Haaren in dem Intimhautbereich, umfassend die wasserfreie Kosmetikzusammensetzung, wobei der Inkontinenzabsorptionsartikel einen Einsatz, eine Einlage oder ein Höschen umfasst.

**13.** Verfahren nach Anspruch 10 und 11, wobei die wasserfreie Kosmetikzusammensetzung einen kosmetischen Wirkstoff umfasst, der ein öllösliches Derivat von Vitamin B5 ist.

**14.** Verfahren nach Anspruch 10 und 12, wobei die wasserfreie Kosmetikzusammensetzung einen kosmetischen Wirkstoff umfasst, der Zinkoxid ist.

15. Verfahren nach einem der vorstehenden Ansprüche, wobei die wasserfreie Kosmetikzusammensetzung einen substantiven Schutzfilm bei Anwendung auf der Haut in dem Intimbereich bereitstellt.

**Revendications**

1. Utilisation d'une composition cosmétique anhydre pour neutraliser les mauvaises odeurs par application topique sur des zones intimes, la composition cosmétique anhydre comprenant :
un solvant volatil, et un ou plusieurs composés complexés ou encapsulés choisis dans le groupe constitué de : mélonal, adoxal, trans-2-hexénal, ligustral, Floral Super, florhydral, 5-méthyl-2-thiophène-carboxaldéhyde, aldéhyde hydratropique, undécanal, 9-undécénal, 10-undécénal, trans-4-décénal, cis-6-nonénal, isocyclocitral, précyclémone b, €-2-(z)-6-nonadiénal, aldéhyde undécylique, méthyl-octyl-acétaldéhyde, aldéhyde laurique, silvial, vanilline, et floralozone dans laquelle la composition cosmétique anhydre est sous la forme d'un bâton solide comprenant une dureté de produit d'au moins 600 grammes-force (telle que mesurée par le procédé décrit dans la description) ; et dans laquelle la composition cosmétique anhydre est dépourvue d'actif anti-transpirant.

2. Utilisation de la composition cosmétique anhydre selon la revendication 1, dans laquelle ledit ou lesdits composés sont encapsulés par de l'amidon.

3. Utilisation de la composition cosmétique anhydre selon l'une quelconque des revendications précédentes, dans laquelle ledit ou lesdits composés sont encapsulés dans des capsules de type réservoir et peuvent être libérés par une action mécanique rompant l'enveloppe de la capsule.

4. Utilisation de la composition cosmétique anhydre selon l'une quelconque des revendications précédentes, dans laquelle le ou les composés sont constitués de complexes de cyclodextrine.

5. Utilisation de la composition cosmétique anhydre selon l'une quelconque des revendications précédentes, comprenant en outre un actif cosmétique, dans laquelle l'actif cosmétique est l'oxyde de zinc.

6. Utilisation de la composition cosmétique anhydre selon les revendications 1 à 4, comprenant en outre un actif cosmétique, dans laquelle l'actif cosmétique est le triacétate de panthényle.

7. Utilisation de la composition cosmétique anhydre selon les revendications 1 à 4 et 6, comprenant en outre un actif cosmétique choisi dans le groupe constitué de l'oxyde de zinc et d'un dérivé de vitamine B5 soluble dans l'huile.

8. Utilisation de la composition cosmétique anhydre selon l'une quelconque des revendications précédentes, dans laquelle la dureté de produit est d'environ 800 grammes-force à environ 1 400 grammes-force.

9. Utilisation de la composition cosmétique anhydre selon l'une quelconque des revendications précédentes, dans laquelle la composition cosmétique anhydre comprend moins de 1 pour cent en poids de propylène glycol et de dipropylène glycol.

10. Procédé de traitement de la peau dans la zone intime pour neutraliser les mauvaises odeurs, le procédé comprenant les étapes consistant à :
frotter une composition cosmétique anhydre qui est dépourvue d'actif anti-transpirant sur la peau et/ou les poils dans une zone de peau intime pour appliquer la composition cosmétique anhydre à celle-ci, la composition cosmétique anhydre comprenant :

une dureté de produit d'au moins 600 grammes-force ; et un solvant volatil, et
un ou plusieurs composés complexés ou encapsulés choisis dans le groupe constitué de : mélonal, adoxal, trans-2-hexénal, ligustral, Floral Super, florhydral, 5-méthyl-2-thiopène-carboxaldéhyde, aldéhyde hydratropique, undécanal, 9-undécénal, 10-undécénal, trans-4-décénal, cis-6-nonénal, isocyclocitral, précyclémone b, €-2-(z)-6-nonadiénal, aldéhyde undécylique, méthyl-octyl-acétaldéhyde, aldéhyde laurique, silvial, vanilline, et floralozone.

11. Procédé selon la revendication 10, comprenant en outre les étapes consistant à (i) raser ou raccourcir les poils de

la peau dans la zone de peau intime, et (ii) après cela, préparer la peau dans la zone de peau intime pour l'application d'une composition cosmétique dont chacune a lieu avant l'étape d'application dans la revendication 10.

**12.** Procédé selon la revendication 10, comprenant en outre l'étape consistant à appliquer un article absorbant pour incontinence contre la peau et/ou les poils dans la zone de peau intime comprenant la composition cosmétique anhydre, l'article absorbant pour incontinence comprenant une protection, un tampon ou un slip.

**13.** Procédé selon les revendications 10 et 11, dans lequel la composition cosmétique anhydre comprend un actif cosmétique qui est un dérivé soluble dans l'huile de la vitamine B5.

**14.** Procédé selon les revendications 10 et 12, dans lequel la composition cosmétique anhydre comprend un actif cosmétique qui est l'oxyde de zinc.

**15.** Procédé selon l'une quelconque des revendications précédentes, dans lequel la composition cosmétique anhydre fournit un film protecteur substantif lors de l'application sur la peau dans la zone intime.

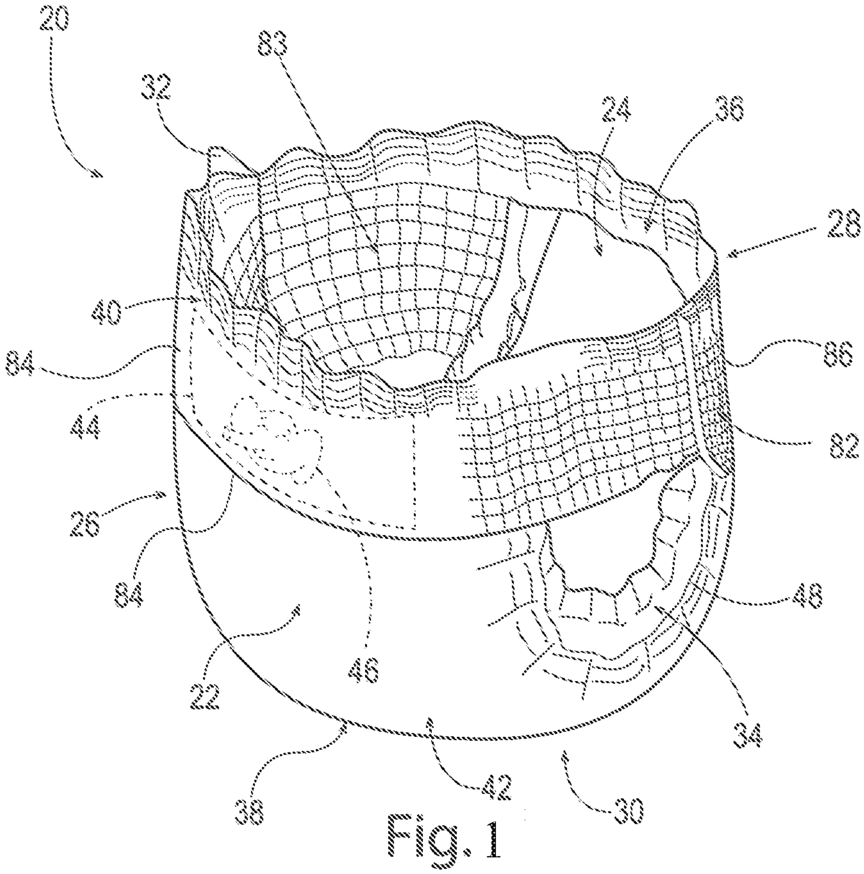


Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0074643 A
- US 20050003980 A1
- US 20040110648 A1
- US 20040092414 A1
- US 20040091445 A1
- US 20040087476 A1
- US 6531444 B
- US 6024943 A
- US 6042792 A
- US 6051540 A
- US 4540721 A
- US 4973422 A
- WO 2005102261 A
- US 20050124530 A1
- US 20050143282 A1
- WO 2003015736 A
- US 2006003913 A1
- US 2012276210 A1
- US 61703587 B
- US 61703616 A
- US 61328949 A
- US 61328954 A
- US 61328962 A
- US 61328967 A
- US 6458754 B1
- US 5571782 A
- US 5543157 A
- WO 2008104690 A2
- US 20080213191 A1
- US 6001377 A, SaNogueira, Jr.
- US 6024942 A, Tanner
- US 6013271 A, Doughty
- US 6013270 A, Hargraves
- US 6013248 A, Luebbe
- US 5976514 A, Guskey
- US 6352688 B, Scavone
- US 5968489 A, Swaile
- US 6835373 B, Kolodzik
- US 5429816 A
- US 5840287 A
- US 4049792 A, Elsnau
- US 2013281948 A1
- US 5019375 A, Tanner
- US 5965113 A, Guskey
- US O6590136 A


### Non-patent literature cited in the description


- Chemical Terminology. 1997
- **TODD et al.** Volatile Silicone Fluids for Cosmetics. *Cosmetics and Toiletries,* 1976, vol. 91, 27-32
- **C. D. VAUGHAN.** Solubility Effects in Product, Package, Penetration and Preservation. *Cosmetics and Toiletries,* October 1988, vol. 103, 47-69
- **C. D. VAUGHAN.** Using Solubility Parameters in Cosmetics Formulation. *J. Soc. Cosmetic Chemists,* September 1988, vol. 36, 319-333